(19) Europäisches Patentamt
European Patent Office
Office européen des brevets

(11) Publication number : **0 331 471 B1**

(12) # EUROPEAN PATENT SPECIFICATION

(45) Date of publication of patent specification : **16.12.92 Bulletin 92/51**

(51) Int. Cl.$^5$ : **A61K 47/00**, C08B 37/02, C08B 31/12, A61K 47/48

(21) Application number : **89302051.1**

(22) Date of filing : **01.03.89**

(54) **High molecular weight prodrug derivatives antiinflammatory drugs.**

(30) Priority : **02.03.88 DK 1101/88**

(43) Date of publication of application : **06.09.89 Bulletin 89/36**

(45) Publication of the grant of the patent : **16.12.92 Bulletin 92/51**

(84) Designated Contracting States : **AT BE CH DE ES FR GB GR IT LI LU NL SE**

(56) References cited :
**EP-A- 0 019 403**
**EP-A- 0 251 905**
**GB-A- 2 041 958**
**JOURNAL OF APPLIED POLYMER SCIENCE, vol. 30, no. 7, July 1985, pages 2761-2778, John Wiley & Sons, Inc. New York, US;N.V. KHUE et al.: "Antiinflammatory polymer-bound steroids for topical applications. I. Synthesis and characterisation"**
**CHEMICAL ABSTRACTS, vol. 78, no. 26, July 2, 1973, page 289, abstract no. 164099a, Columbus, Ohio, US**
**CHEMICAL ABSTRACTS, vol. 94, no. 24, June 15, 1981, page 350, abstract no. 197486h, Columbus, Ohio, US; J.M. WHITELEY et al.: "The biochemical properties of carrierbound methotrexate"**

(73) Proprietor : **Larsen, Claus Selch**
**15, Hulegaardsvej**
**DK-4320 Lejre (DK)**
Proprietor : **Johansen, Marianne**
**Stockholmsgade 21**
**DK-2100 Copenhagen 0 (DK)**
Proprietor : **Harboe, Elin**
**Skjalm Hvidesgade 11**
**DK-1728 Copenhagen V (DK)**
Proprietor : **Kurtzhals, Peter**
**Akelejehaven 53**
**DK-2630 Tasstrup (DK)**
Proprietor : **Olesen, Henning Peter**
**Erdalsvej 3**
**DK-2600 Glostrup (DK)**

(72) Inventor : **Larsen, Claus Selch**
**15, Hulegaardsvej**
**DK-4320 Lejre (DK)**
Inventor : **Johansen, Marianne**
**Stockholmsgade 21**
**DK-2100 Copenhagen 0 (DK)**
Inventor : **Harboe, Elin**
**Skjalm Hvidesgade 11**
**DK-1728 Copenhagen V (DK)**
Inventor : **Kurtzhals, Peter**
**Akelejehaven 53**
**DK-2630 Tasstrup (DK)**
Inventor : **Olesen, Henning Peter**
**Erdalsvej 3**
**DK-2600 Glostrup (DK)**

(74) Representative : **Andersen, Henrik Rastrup et al**
**c/o Plougmann & Vingtoft Sankt Annae Plads 11 P.O. Box 3007**
**DK-1021 Copenhagen K (DK)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to novel high molecular weight prodrug forms of drugs useful in the treatment and the relief of pain of conditions characterized by inflammation, such as rheumatism, arthritis, gout and ulcerative colitis, to methods for preparing the prodrug forms, to pharmaceutical compositions containing such prodrug forms, and to methods for using the prodrug forms.

For purposes of this specification, the term "prodrug" denotes a derivative of a known and proven antiinflammatory agent (e.g. naproxen, ibuprofen, ketoprofen, hydrocortisone, 5-aminosalicylic acid, methylprednisolone etc.) which derivative, when administered to warm-blooded animals, including humans, is converted into the proven drug. The enzymatic and/or chemical hydrolytic cleavage of the compounds of the present invention occurs in such a manner that the proven drug form (parent drug compound) is released, and the moiety or the moieties split off remain nontoxic or are metabolized so that nontoxic metabolites are produced.

In these novel prodrug forms the antiinflammatory drug compounds have been linked covalently to certain biodegradable polysaccharide derivatives either directly through ester linkages or by intercalating between the drug and the polysaccharide carrier a suitable spacer arm. After parenteral administration these novel prodrug forms combine a prolonged duration of activity, by slowly releasing the active antiinflammatory drug at the site of administration, with a desirably high stability in aqueous solution in the pH range 3 - 5 in vitro. Due to the molecular size of the polysaccharide carrier molecule the new prodrug forms are further characterized by a restricted mobility in vivo, thus allowing the active drug compound to be regenerated in a localized manner at the administration site in the vicinity of the diseased tissue. After oral administration to warm-blooded animals of such prodrug conjugates prolonged and localized release of the parent active agent takes place in the terminal ileum and in the colon effected by glucosidases and hydrolases situated in that part of the GI-tract. Besides providing selective delivery to the terminal ileum and the colon after oral administration, the prodrug conjugates give rise to therapeutically effective and constant concentration of the released active drugs in the blood over an extended period of time. Furthermore the new prodrug forms are endowed with a desirably high water-solubility at pH 3-5 in comparison to the parent antiinflammatory drug compounds. It is still a further property of the prodrug derivatives that they exhibit a feasible tissue compatibility.

It is well known that a wide variety of drug compounds are used in the management of disorders characterized by inflammation. These drug compounds include non-steroidal antiinflammatory drugs (NSAIDs) which in this context are defined as derivatives of anthranilic acid, phenylalkanoic acids and indomethacin, such as naproxen, ketoprofen, ibuprofen and diclofenac; corticosteroids such as hydrocortisone, prednisolone, methylprednisolone and triamcinolone; antimalarials such as hydroxychloroquine; immunosuppressives such as methotrexate and melphalan; 5-aminosalicylic acid as well as other drug compounds having diverse biological properties and structures.

Disorders characterized by inflammation, which frequently are treated by the above mentioned drug compounds, include:

*Synovitis*

a. Adult and juvenile rheumatoid arthritis

b. Other collagen vascular disorders (e.g. systemic lupus erythematosus, mixed collective tissue disease syndrome)

c. Crystal-induced arthropaties (gout, pseudogout)

d. Seronegative spondyloarthropathies (peripheral joint involvement of ankylosing spondylitis, psoriatic arthritis, Reiter's syndrome, inflammatory bowel disease)

e. Knee synovitis following hip arthroplasty

f. Acute trauma

*Synovial cyst of Heberden's Nodes*

*Adhesive capsulitis (frozen shoulder)*

*Shoulder-hand syndrome*

*Popliteal and antecubial cysts*

*Tendinitis*

a. Supraspinatus, bicipital, wrist extensor, De Quervain's syndrome, flexor carpi radialis and ulnaris, digital flexor (trigger finger),

Achilles, semimembranosus

*Bursitis*

a. Subacromial, coracoid, olecranon, trochanteric, anserine, prepatellar, infrapatellar, retiocalcaneal

*Carpal,Guyon, and tarsual tunnel syndrome*

*Epicondylitis*

*Plantar fascitis*

*Temporomandibular joint syndromes*
*Osteoarthritis*
a. Knee and inflammatory interphalangeal joint synovitis
b. First metacarpophalangeal, carpometacarpal, and metatarsophalangeal joints
c. Lumbar facet arthropathy
*Ganglia*
*Fibrositic trigger points*
*Low back syndrome*
*Tietze's syndrome*
*Costochondrosis*
*Xiphoiditis*
*Dupuytren's contracture*
*Rheumatoid nodules*
*Episacroiliac lipomate (Stockman's nodules)*
*Hand swelling of mixed connective tissue disease*
*Soft tissue flexion contractures (recent)*
*Sciatica*
*Acute lumbar disc prolapse*
*Ulcerative colitis*
*Crohn's disease*

Other indications for the above mentioned antiinflammatory agents will be apparent for those skilled in the art.

In the management of inflammatory disorders, a medicinal need exists for new pharmaceutical parenteral formulations of antiinflammatory drugs, which after administration to warm-blooded animals locally in the vicinity of the inflamed tissue (for example intra-articular administration) provide liberation of the active inflammatory agent with a well-defined rate (*controlled release*) over an extended period of time (*prolonged duration of action*) at the site of administration (*localized drug action*). This need exists because conventional formulations of antiinflammatory drugs used hitherto in the treatment of inflammatory disorders suffer from several drawbacks. After oral administration of NSAIDs, only a small amount of the instilled dose gains access to the inflamed tissue (for example to inflamed joints) (Gallo et al. (1986); Mäkela et al. (1981)). Since the duration of activity of NSAIDs are limited, frequent administration of massive amounts of NSAIDs is therefore necessary in order to maintain therapeutically effective concentrations of NSAIDs locally at the diseased site. This administration pattern in turn results in undesirable side-effects such as microvascular blood loss from the GI tract and gastric ulcers (Baker and Rabinowitz (1986) and references cited therein).

Furthermore a low-level NSAID therapy, provided by localized and prolonged duration of action, is strongly needed since most side-effects associated with NSAID therapy are dose-related. This is the case for the above mentioned damage of the gastrointestinal mucosa, which in addition is systemic in nature (Baker and Rabinowitz (1986); Bjarnason et al. (1984)). Another serious dose-dependent side-effect is the significant mental status change of elderly while taking a variety of NSAIDs (Baker and Rabinowitz (1986) and references cited therein).

Microcrystalline aqueous suspensions of corticosteroids are available for intra-articular administration. The crystals are retained within the joint and dissolve slowly producing a sustained antiinflammatory effect. However, a significant amount of the instilled dose leaches to the systemic circulation in an uncontrolled manner producing serious side-effects such as suppression of endogenous cortisol production (Hunneyball (1986); Gray and Gottlieb (1983)). In addition the crystal preparation, per se, give rise to local flare reactions due to the physical nature of the drug formulation (Gray and Gottlieb (1983); Hunneyball (1986)).

Since antiinflammatory drug therapy is associated with several and severe side-effects, the development of drug formulations to achieve a localized, low-level, prolonged-effect therapy would represent a major advantage (Hunneyball (1986) and references cited therein). The need for drug formulations with these desirable attributes has been generally recognized (Ratcliffe et al. (1987)). Apart from the application of corticosteroid suspension, attempts to achieve local and prolonged duration of action after intra-articular injection include incorporation of antiinflammatory drugs in liposomes (Dingle et al. (1978)) and in microspheres (Ratcliffe et al. (1984); Ratcliffe et al. (1987)). These colloidal approaches suffer from several drawbacks and differ considerably from the approach and the compounds of the present invention.

Human inflammatory bowel diseases such as ulcerative colitis and Crohn's disease are currently treated by oral administration of prednisolone or sulfasalazine. The latter drug is assumed to be cleaved in the lower bowel by anaerobic bacteria to yield the therapeutically active 5-aminosalicylic acid. Oral therapy by using formulations of these drugs suffers from several drawbacks mainly due to the non-specific absorption of the drugs

along the gastrointestinal tract (Thomas et al. (1985); Brown et al. (1983)). Consequently, in order to obtain effective concentrations of the drugs at the diseased site, high doses have to be given which in turn leads to severe local as well as systemic side effects. Thus, the limitations to the use of sulfasalazine are for example the development of adverse gastrointestinal, hematological, and generalized side effects, or more serious reactions, including agranulocytosis, toxic epidermal necrolysis, parestesia, hepatotoxicity, pancreatitis, pulmonary disease and male infertility (Brown et al. (1983)). High molecular weight prodrugs of 5-aminosalicylic acid by using synthetic macromolecular carriers have been synthesized with the aim to transport the active agent selectively to the colon (US patent 4,190,716, US patent 4,298,595). However, regeneration of 5-aminosalicylic acid from the latter prodrugs in vivo was poor.

In view of the foregoing, it is quite obvious that a serious need exists for improved parenteral and oral formulations of antiinflammatory drugs which will overcome the aforementioned disadvantages. From the foregoing, it also appears that successful high molecular weight prodrug forms of antiinflammatory drugs should be retained at the site of administration or should deliver the parent drug selectively to the inflamed tissue (for example within a joint cavity), should be tissue compatible and finally should lead to a controlled release and prolonged duration of action of antiinflammatory drugs at the diseased site.

It is an object of the present invention to provide such derivatives of antiinflammatory drugs which are prodrugs designed to cleave in such a manner as to enable the original parent drug form to be released at its target site or sites of activity, while the remaining cleaved moiety is nontoxic and/or is metabolized in a nontoxic fashion.

It is another object of the present invention to provide novel high molecular weight prodrug types of antiinflammatory drugs characterized by possessing prolonged duration of activity by slowly and in a controlled and predictable manner releasing the active antiinflammatory drug in vivo. The prodrug forms are further characterized by exhibiting a desirably high stability in aqueous media in the pH range 3 - 5 in vitro.

It is a further object of the present invention to provide novel bioreversible derivatives for antiinflammatory drugs which derivatives, when administered intra-articularly to warm-blooded animals, remain in the joint cavity or by endocytosis are taken up by the inflammatory cells in the synovium, thus combining localized drug action with a sustained release of the active drug compound.

It is still another object of this invention to provide novel prodrug forms of antiinflammatory drugs which derivatives, when given to warm-blooded animals by local parenteral administration in the vicinity of other tissues characterized by inflammatory disorders, provide localized and prolonged drug action at the site of administration.

It is still another object of this invention to provide novel prodrug forms of antiinflammatory drugs which derivatives, when given orally to warm-blooded animals, regenerate the parent drug compound selectively in the terminal ileum and in the colon over an extended period of time (localized and sustained release formulations).

It is yet another object of this invention to provide novel prodrug forms of antiinflammatory drugs which derivatives, when given orally to warm-blooded animals, result in sufficiently high and constant concentration of the released active drug in the blood over an extended period of time (sustained release formulations).

It is yet another object of this invention to provide high molecular weight prodrug types of antiinflammatory drugs which derivatives, when administered to warm-blooded animals, elicit the bio-affecting/pharmacological response characteristic of the drugs from which they are derived, yet which are characterized in being less irritating to the tissues surrounding the administration site.

Other objects, features and advantages of the invention will be apparent to those skilled in the art.

The foregoing objects, features and advantages are provided by the novel compounds of formula 1

$$PS - O - A - (CH_2)_n - B - D \qquad (1)$$

wherein PS - O represents an alkoxide residue of any of the free hydroxy groups of a polysaccharide derivative (PS - OH) as defined below,

A is a carbonyl group or absent,

n is zero or a positive integer from 1 to 14,

B is oxygen, a carbonyl group, NR wherein R is hydrogen or straight or branched-chain $C_{1-8}$ alkyl, or B is absent, and

D is

(i)

$$R_1\text{-CO-} \qquad (1_1)$$

wherein $R_1$ - CO - represents the acyl residue of a carboxylic acid drug or medicament ($R_1$ - COOH) used in the treatment of inflammatory disorders;

(ii)

$R_2$-O- $(1_2)$

wherein $R_2$-O- refers to the C-21 alkoxide residue of a known antiinflammatory steroid ($R_2$-OH) or an alkoxide residue of any other drug or medicament used in the treatment of inflammatory disorders and which containins a hydroxy functional group ; with the proviso that when PS-O is the alkoxide residue of dextran, A is absent, n is O and B is absent, then R-CO- is different from the acyl residue of acetylsalicyclic acid; and with the further proviso that compounds having peroxo moities

and nontoxic pharmaceutically acceptable acid addition salts thereof;

and nontoxic pharmaceutically acceptable cation salts thereof.

In the present context the term "polysaccharide" applies to carbohydrate polymers that contain periodically repeating structures in which the dominant interunit linkage is of the O-glycosidic type. In the present invention the macromolecular carriers used for the antiinflammatory drugs are polysaccharides such as dextran and derivatives thereof such as carboxymethyl dextran and diethylaminoethyl dextran, glycogen, pullullan, agarose, cellulose, chitosan, chitin and carrageenan. In the present application, PS-OH signifies any such polysaccharide carrier compound.

The polymer backbone of the polysaccharides and their derivatives described in this invention differ slightly in chemical structure. However, prodrug conjugates containing identical ligands but are derived from various of the aforementioned polysaccharides behave similarly to the dextran conjugates of the antiinflammatory drugs defined in formula 1 as regards condition of synthesis, physico-chemical properties and biological activity. Thus, this invention includes the use of dextran as well as the other aforementioned polysaccharide derivatives as feasible macromolecular carriers for antiinflammatory drugs.

The dextrans are high molecular weight polysaccharides made up of α-D-anhydroglucopyranosidic units and characterized in that the linkage between the monomeric units are of both α-1,6 and non-α-1,6 type, at least 50% of these linkages being of the α-1,6 type.

A striking feature of the dextrans is the wide variations they exhibit with respect to their physical and structural properties including molecular weight, molecular weight distribution, molecular structural repeating α-1,6 to non-α-1,6 linkages ratio, and the water sensitivity. As to the latter property, while the so-called "native dextrans", being hydroxylbearing substances, are hydrophilic, some of the dextrans are readily soluble in water whereas others are difficultly soluble in water, are initially swollen thereby and only ultimately, if at all, completely dissolved therein.

A wide variety of dextrans may be used in practicing this invention, which, as stated above, concerns the use of dextrans as carriers for antiinflammatory drugs. The dextran used may have a molecular weight of from 5,000 to 150 x $10^6$ as determined by light scattering measurements, a molecular structural repeating α-1,6 to non-α-1,6 linkages ratio of from 1.9:1 to 30:1; a polydispersity of from 1.1 to 10 as defined as the ration $M_w/M_n$, where the $M_w$ and $M_n$ refer to the weight average molecular weight and the number average molecular weight, respectively; and be soluble or substantially insoluble in water depending on the use for which the specific drug carrier is intended.

The dextrans may be obtained by various methods. They may be synthesized from sucrose by enzyme action in the presence or substantially absence of bacteria. For example, an aqueous nutrient medium containing sucrose, particularly nitrogenous compounds and certain inorganic salts, may be inoculated with a culture of an appropriate microorganism such as those of the *Leuconostoc mesenteroides* and *L. dextranicum* types, and incubated at the temperature most favourable to the growth of the microorganism until maximum dextran production is attained. This is synthesis of the dextran from sucrose by the so-called "whole culture" method, i.e., the synthesis is effected by enzyme action in the presence of the bacteria and cellular debris. Or the culture obtained by cultivating the Leuconostoc bacterium may be filtered to isolate the enzyme (dextransucrase) which occurs in the filtrate. The filtrate, usually after dilution to predetermined enzyme potency, may be mixed with an aqueous sucrose solution, and the mixture may be allowed to stand under controlled conditions of pH and temperature until the dextran is synthesized. The enzyme may be separated from the filtrate and used in powdered condition or in the form of an aqueous solution, usually the latter. This is dextran synthesis by enzyme action in the substantial absense of bacteria and cellular debris.

The dextran obtained initially by these procedures is so-called "native" dextran which normally has a very high average molecular weight, calculated to be in the millions. It may be precipitated from the medium in which it is synthesized by the addition of an organic liquid which is a non-solvent for the dextran. The non-solvent, or precipitant, may be a water-miscible aliphatic alcohol, e.g. methanol, ethanol or isopropanol, or a ketone such as acetone, or dioxane. The precipitated dextran may be purified and dried to a substantially white mass which may be reduced to powdered condition for use in the synthesis.

Native or high molecular weight dextran may be hydrolyzed under acid or neutral conditions, or by enzyme action to a molecular weight lower than that of the native material. Thus "clinical" dextran has an average molecular weight of from 20,000 or 200,000. In "clinical" dextran production, when the desired molecular weight

is obtained by hydrolysis or cleavage of the native material, it is usual to isolate the "clinical" product from the hydrolysate by fractional precipitation according to which, by successive addition of increasing amounts of water-miscible alcohol or ketone, the highest molecular weight fraction is first thrown down and separated, and the desired or intermediate molecular fraction is then precipitated and recovered. This procedure leaves a supernatant containing dextran the average molecular weight of which is below the "clinical" range, and the supernatant is usually discarded as waste. The different dextran fractions may also be isolated from the hydrolyzate by fractional solution methods involving the use of the precipitant in conjunction with a dextran solvent, usually water. It may be noted, here, that when the dextran synthesis is effected by the action of the enzyme on sucrose in the absence of bacteria, it is possible to carry out the synthesis under conditions such as to favour the production of dextran of relatively low molecular weight in at least preponderant proportion. It is possible, therefore, as is now known, to obtain relatively low average molecular weight dextran by direct enzymatic synthesis from sucrose. Furthermore, after repeated gelfiltration of the isolated dextran fractions, using for example the Sephadex® series, dextran products with a polydispersity as low as 1.1 may be obtained. When dextran is synthesized from sucrose by enzyme action, in the presence or substantial absence of bacteria and cellular debris, the water-sensitivity of the native dextran obtained is influenced by the microorganism cultivated to obtain the culture, or enzyme isolatable therefrom, introduced into the sucrose-bearing medium in which the dextran is to be synthesized. Thus, native dextrans synthesized by the use of the microorganisms bearing the following NRRL (Northern Regional Research Laboratories) classification, or their enzymes, are quite readily soluble in water: *Leuconostoc mesenteroides* B-512, B-1146, B-119, and B-1196. These dextrans are, usually, smooth, lustrous, elastic gums which are quite readily soluble in water to give clear or substantially clear solutions.

The native dextrans from the microorganisms (or their enzymes) (NRRL) *Leuconostoc mesenteroides* B-742, B-1191, B-1208, and B-1216, and from *Streptobacterium dextranicum* B-1254 are, generally speaking, rather rough, dull, non-elastic gums which may be regarded as relatively insoluble in water but which are water-swellable and go into solution in water under heating and stirring to give viscous solutions that are somewhat turgid.

A third group of native dextrans is represented by and includes those obtained from microorganisms (or their enzymes) bearing the NRRL classifications: *Leuconostoc mesenteroides* B-1120, B-1144, B-523, and *Betabacterium vermiforme* B-1139. These dextrans are generally more or less flocculent gums, which are swellable by water but which are, for all practical purposes, substantially insoluble therein.

Polysaccharides including dextrans contain a huge number of hydroxy groups available for covalent attachment of organic substances, hereinafter called "ligands". In dextran predominantly the hydroxy groups of the monomeric α-D-glucose unit at the position C-2, C-3, C-4 are available for ligand fixation, but also the free hydroxy groups at the position C-6 of the terminal α-D-glucose units in the main chains as well as the side chains of dextran may be used for establishment of dextran-ligand bonds.

With regard to establishment of covalently linked ligands the polysaccharide hydroxy groups at the positions C-2, C-3, C-4 and C-6 differ only slightly in reactivity (de Belder and Norrman (1968); Larsen and Johansen (1985)). Furthermore in case of ligand attachment accomplished through polysaccharide ester formation the proportion in which ester bonds are formed at the C-2, C-3, C-4 and C-6 position is thermodymanically determined due to acyl migration (Casinovi et al. (1974)). Consequently no single hydroxy group of the monomeric carbohydrate unit is exclusively preferred for ligand fixation. Although the same ligand or different ligand types theoretically might occupy all the hydroxy groups of one single monomeric carbohydrate unit of the polysaccharide chain it is much more likely that independently of the synthesis conditions the covalently attached ligands will be distributed uniformly along the polysaccharide chains (Larsen and Johansen (1985)).

In the present context, the $C_{1-8}$ alkyl may be straight or branched, such as methyl, ethyl, propyl, isopropyl, butyl, tert. butyl, pentyl, hexyl, heptyl, or octyl.

The term "nontoxic pharmaceutically acceptable acid addition salts" as used herein generally includes the nontoxic acid addition salts of compounds of formula 1, formed with nontoxic inorganic or organic acids. For example, the salts include those derived from inorganic acids such as hydrochloric, hydrobromic, sulphuric , sulphamic, nitric and phosphoric; and salts with organic acids such as acetic, propionic, succinic, fumaric, maleic, tartaric, citric, glycolic, lactic, stearic, malic, pamoic, ascorbic, phenylacetic, benzoic, glutamic, salicylic and sulphanilic.

The term "nontoxic pharmaceutically acceptable cation salts" as used herein generally includes the nontoxic cation salts of compounds of formula 1, formed with nontoxic inorganic or organic bases. For example, the salts include those derived from cations such as potassium, sodium, calcium, magnesium, zinc, chlorprocaine, diethanolamine, ethylendiamine, meglumine, procaine, diethylamine, piperazine and tromethamine.

As stated above, D in the formula 1 can represent the acyl residue $R_1$-CO- (in formula $1_1$) of any drug,

pharmaceutical or medicament ($R_1$-COOH), useful in the treatment of inflammatory disorders, having one or more carboxylic acid functions. Examples of drugs or pharmaceuticals from which the instant high molecular weight prodrugs are derived include but are not limited to:

*a*. Non-steroidal antiinflammatory agents like:

Sulindac: (z)-[5-fluoro-2-methyl-1-(4-methylsulphinylbenzylidene)inden-3-yl]acetic acid
Indometacin: 1-(4-chlorobenzoyl)-5-methoxy-2-methyl-1H-indole-3-acetic acid
Naproxen: (+)-2-(6-methoxy-2-napthyl)propionic acid
Fenoprofen calcium: calcium (±)-2-(3-phenoxyphenyl)propionate dihydrate
Ibuprofen: 2-(4-isobutylphenyl)propionic acid
Ketoprofen: 2-(3-benzoylphenyl)propionic acid
Indoprofen: 2-[4-(1-oxoisoindolin-2-yl)phenyl]-propionic acid
Diflunisal: 5-(2,4-difluorophenyl)salicylic acid
Tolmetin sodium: sodium (1-methyl-5-p-toluoylpyrrol-2-yl)acetate dihydrate
Flurbiprofen: 2-(2-fluorobiphenyl-4-yl)propionic acid
Diclofenac sodium: sodium [2-(2,6-dichloroanilino)phenyl]acetate
Mefenamic acid: N-(2,3-xylyl)anthranilic acid
Flufenamic acid: N-(ααα-trifluoro-m-tolyl)anthranilic acid
Meclofenamic acid: N-(2,6-dichloro-m-tolyl)-anthranilic acid
Fenclozic acid: 2-(4-chlorophenyl)-4-thiazoleacetic acid
Alclofenac: (4-allyloxy-3-chlorophenyl)acetic acid
Bucloxic acid: 3-(3-chloro-4-cyclohexylbenzoyl)-propionic acid
Suprofen: α-methyl-4-(2-thienylcarbonyl)benzeneacetic acid
Fluprofen: 3′-fluoro-α-methyl-[1,1′-biphenyl]-4-acetic acid
Cinchophen: 2-phenylquinoline-4-carboxylic acid
Pirprofen: 2-[3-chloro-4-(3-pyrrolin-1-yl)phenyl]propionic acid
Cinmetacin: 5-methoxy-2-methyl-1-(1-oxo-3-phenyl-2-propenyl)-1H-indole-3-acetic acid
Acemetacin: 1-(4-chlorobenzoyl)-5-methoxy-2-methyl-1H-indole-3-acetic acid carboxymethyl ester
Ketorolac: (±)-5-benzoyl-2,3-dihydro-1H-pyrrolizine-1-carboxylic acid
Clometacin: [3-(4-chlorobenzoyl)6-methoxy-2-methyl-indol-1-yl]acetic acid
Ibufenac: 4-(2-methylpropyl)-benzeneacetic acid
Tolfenamic acid: N-(3-chloro-o-tolyl)anthranilic acid
Fenclofenac: [2-(2,4-dichlorophenoxy)phenyl]acetic acid
Prodolic acid: 1,3,4,9-tetrahydro-1-propyl-pyrano[3,4-b]indole-1-acetic acid
Clonixin: 2-(3-chloro-o-toluidino)nicotinic acid
Flutiazin: 8-(trifluoromethyl)-10H-phenothiazine-1-carboxylic acid
Flufenisal: 4-(acetyloxy)-4′-fluoro-[1,1′-biphenyl]-3-carboxylic acid
O-(Carbamoylphenoxy)acetic acid
Zomepirac sodium: sodium [5-(4-chlorobenzoyl)-1,4-dimethylpyrrol-2-yl]acetate dihydrate
Niflumic acid: 2-(ααα-trifluoro-m-toluidino)nicotinic acid
Lonazolac: 3-(4-chlorophenyl)-1-phenyl-1H-pyrazole-4-acetic acid
Fenbufen: 4-(biphenyl-4-yl)-4-oxobutyric acid
Carprofen: (±)-6-chloro-α-methyl-9H-carbazole-2-acetic acid
Tiaprofenic acid: 2-(5-benzoyl-2-thienyl)propionic acid
Loxoprofen: α-methyl-4-[2-oxocyclopentyl)methyl]-benzeneacetic acid
Etodolac: 1,8-diethyl-1,3,4,9-tetrahydro-pyrano[3,4-b]indole-1-acetic acid
Alminoprofen: α-methyl-4[(2-methyl-2-propenyl)amino]-benzeneacetic acid
2-(8-Methyl-10,11-dihydro-11-oxodibenz[b,f]oxepin-2-yl)propionic acid
4-Biphenylacetic acid

*b*. 4-Quinolone antibiotics like:

Ciprofloxacin: 1-cyclopropyl-6-fluoro-1,4-dihydro-4-oxo-7-(1-piperazinyl)-3-quinolinecarboxylic acid
Norfloxacin: 1-ethyl-6-fluoro-1,4-dihydro-4-oxo-7-(1-piperazinyl)-3-quinolinecarboxylic acid
Acrosoxacin: ethyl-1,4-dihydro-4-oxo-7-(4-pyridyl)quinoline-3-carboxylic acid
Pipemidic acid: 8-ethyl-5,8-dihydro-5-oxo-2-(pyrrolidin-1-yl)pyrido[2,3-d]pyrimidine-6-carboxylic acid
Nalidixic acid: 1-ethyl-1,4-dihydro-7-methyl-4-oxo-1,8-naphthyridine-3-carboxylic acid
Enoxacin: 1-ethyl-6-fluoro-1,4-dihydro-4-oxo-7-(1-piperazinyl)-1,8-naphthyridine-3-carboxylic acid
Ofloxacin: (±)-9-fluoro-2,3-dihydro-3-methyl-10-(4-methyl-1-piperazinyl)-7-oxo-7H-pyrido[1,2,3-de]-1,4- benzoxazine-6-carboxylic acid

Oxolinic acid: 5-ethyl-5,8-dihydro-8-oxo-1,3-dioxolo[4,5-g]quinoline-7-carboxylic acid
Flumequine: 9-fluoro-6,7-dihydro-5-methyl-1-oxo-1H, 5H-benzo[ij]quinolizine-2-carboxylic acid
Cinoxacin: 1-ethyl-1,4-dihydro-4-oxo-[1,3]dioxolo[4,5-g]cinnoline-3-carboxylic acid
Piromidic acid: 8-ethyl-5,8-dihydro-5-oxo-2-(pyrrolidin-1-yl)pyrido[2,3-d]pyrimidine-6-carboxylic acid
Pefloxacin: 1-ethyl-6-fluoro-1,4-dihydro-7-(4-methyl-1-piperazinyl)-4-oxo-3-quinolinecarboxylic acid

*c*. Various other bio-affecting carboxylic acid agents:

Penicillamine: (-)-β,β-dimethylcysteine
5-aminosalicylic acid
6-Aminocaproic acid
Methotrexate: 4-amino-10-methylfolic acid
Sodium cromoglycate: disodium 4,4′-dioxo-5,5′-(2-hydroxytrimethylenedioxy)di(4H-chromene-2-carboxylate)
Chlorambucil: 4-[4-bis(2-chloroethyl)amino-phenyl]butyric acid
Melphalan: 4-bis(2-chloroethyl)amino-L-phenylalanine
All-trans-retinoic acid
13-cis-retinoic acid
Salazosulfapyridine: 4-hydroxy-4′-(2-pyridylsulphamoyl)azobenzene-3-carboxylic acid
Azodisal sodium: disodium 3,3′-azobis[6-hydroxy]-benzoic acid
Gold sodium thiomalate
Furosemide: 4-chloro-N-fufuryl-5-sulfamoylanthranilic acid

As stated above, D in the formula 1 can also represent a C-21 alkoxide residue $R_2$-O- (in formula $1_2$) of a known antiinflammatory steroid ($R_2$-OH) or an alkoxide residue of any other drug or medicament containing a hydroxy functional group, which is used in the management of inflammatory disorders. Examples of drugs or pharmaceuticals from which the instant high molecular weight prodrugs are derived include but are not limited to:

*d*. Antiinflammatory steroids like:

Hydrocortisone: 11β,17α,21-trihydroxypregn-4-ene-3,20-dione
Betamethasone: 9α-fluoro-16β-methylprednisolone
Dexamethasone: 9α-fluoro-16α-methylprednisolone
Prednisolone: 11β,17α,21-trihydroxypregna-1,4-diene-3,20-dione
Triamcinolone: 9α-fluoro-16α-hydroxyprednisolone
Fluocortolone: 6α-fluoro-11β,21-dihydroxy-16α-methyl-pregna-1,4-diene-3,20-dione
Cortisone: 17α,21-dihydroxypregn-4-ene-3,11,20-trione
Fludrocortisone: 9α-fluorohydrocortisone
Chloroprednisone: 6α-6-chloro-17,21-dihydroxy-pregna-1,4-diene-3,11,20-trione
Flumethasone: 6α,9α-difluoro-11β,17α,21-trihydroxy-16α-methylpregna-1,4-diene-3,20-dione
Fluprednisolone: 6α-fluoroprednisolone
Meprednisone: 16β-methylprednisone
Methylprednisolone: 6α-methylprednisolone
Paramethasone: 6α-fluoro-16α-methylprednisolone
Prednisone: 1,2-dehydrocortisone
Amcinafide: [11β,16α(R)]-9-fluoro-11,21-dihydroxy-16,17-[(1-phenylethylidene)bis(oxy)]-pregna-1,4-diene-3,20-dione
Clocortolone: (6α,11β,16α)-9-chloro-6-fluoro-11,21-dihydroxy-16-methyl-pregna-1,4-diene-3,20-dione
Desonide: 16-hydroxyprednisolone 16,17-acetonide
Desoximetasone: 9α-fluoro-11β,21-dihydroxy-16α-methylpregna-1,4-diene-3,20-dione
Flunisolide: (6α,11β,16α)-6-fluoro-11,21-dihydroxy-16,17-[(1-methylethylidene)bis(oxy)]-pregna-1,4-diene-3,20-dione
Fluocinolone acetonide: 6α,9α-difluoro-16α-hydroxyprednisolone acetonide
Triamcinolone acetonide: 9α-fluoro-11β,21-dihydroxy-16α,17α-isopropylidenedioxypregna-1,4-diene-3,20-dione
Betamethasone 17-benzoate
Betamethasone 17-valerate

*e*. Various other bio-affecting hydroxy group containing agents:

1-Aurothio-D-glucopyranose
Hydroxychloroquine: 2-[N-[4-(7-chloro-4-quinolinylamino)pentyl]-N-ethylamino]ethanol sulphate
Amodiaquin: 4-(7-chloro-4-quinolinylamino)-2-(diethylaminomethylphenol dihydrochloride dihydrate

Quinine: (8α,9R)-6′-methoxy-cinchonan-9-ol

All of the above compounds are known in the art in the acid or salt form.

While all of the compounds encompassed by the formula 1 essentially satisfy the objectives of the present investigation, preferred compounds include those derived from the following compounds:

Sulindac
Naproxen
Fenoprofen
Ibuprofen
Ketoprofen
Indoprofen
Flurbiprofen
Mefenamic acid
Flufenamic acid
Meclofenamic acid
Fluprofen
Fenclofenac
Lonazolac
Fenbufen
Carprofen
Loxoprofen
5-aminosalicylic acid
Salazosulfapyridine
Azodisal sodium
Penicillamin
Chlorambucil
Melphalan
Gold sodium thiomalate
Furosemide
Hydrocortisone
Betamethasone
Dexamethasone
Prednisolone
Triamcinolone
Methylprednisolone
Triamcinolone acetonide
Aurothioglucose
Hydroxychloroquine
Amodiaquin
Quinine

Particularly preferred compounds of this invention include those wherein the acyl residue $R_1$-CO- is derived from one of the preferred acids named above, n is zero and A and B are absent. Furthermore, particularly preferred compounds include those wherein the alkoxy residue $R_2$ - O is derived from one of the preferred bioaffecting alcoholic drug compounds named above, A and B are carbonyl groups, n is 2, 3, 4, and PS - O is defined in connection with the general formula 1.

The especially preferred compounds are those particularly preferred compounds in which the polysaccharide carrier (PS - OH) is dextran or hydroxyethyl-starch of molecular weight in the range 40,000 - 5,000,000. The degree of substitution (DS) of the high molecular weight prodrugs are in the range 0.1 - 35%, where DS is defined as the percentage of mg ligand released per mg of the high molecular weight prodrug.

Due to the considerable range of variation in the molecular weight of the drug molecule that can be attached to the polymer, it is advantageous to express the degree of substitution as the percentage of fraction of the free hydroxy groups in the polymer that has been bound to the moiety -A-$(CH_2)_n$-B-D in formula I. Since it is desirable that the compounds of formula I are soluble in water, the maximum useful degree of substitution will to a certain extent depend on the hydrophilic/lipophilic properties of the drug-containing moiety attached to the hydroxy group. Thus, the degree of substitution may be up to 1 of every 5 hydroxy groups, such as up to 1 out of every 10, for example up to 1 out of every 20, e.g. up to 1 out of every 30, alternatively up to 1 out of every 40, in some cases up to 1 out of every 50 hydroxy groups.

*Dosage forms and dose*

The high molecular weight prodrug compounds of formula 1 of the present invention can be used in the treatment and the relief of pain of any condition characterized by inflammation.

The prodrug compounds of formula 1 are designed to be administered parenterally in dosage forms or formulations containing conventional, nontoxic pharmaceutically acceptable carriers and adjuvants including microspheres and liposomes. The formulation and preparation of any of this spectrum of formulations into which the subject prodrugs can be disposed is well-known to those skilled in the art of pharmaceutical formulation. Specific formulation can, however, be found in the text entitled "Remington's Pharmaceutical Sciences", Sixteenth Edition, Mack Publishing Company, 1980.

The pharmaceutical compositions containing the active ingredient are in the form of a sterile injection. To prepare the preferred compositions of this invention, the prodrugs are dissolved or suspended in a parenterally acceptable liquid vehicle. Among the acceptable vehicles and solvents that may be employed are water, water adjusted to pH of from 3.5 to 5.0 by addition of an appropriate amount of 0.1 N hydrochloric acid, 1,3-butanediol, Ringer's solution and isotonic sodium chloride solution. The aqueous formulation may also contain one or more preservatives, for example methyl, ethyl or n-propyl p-hydroxybenzoate. The preferred routes of administration are intra-articular, subcutaneous, intra-muscular and extra-dural.

The prodrug compounds of formula 1 are further designed to be administered orally in dosage forms or formulations such as tablets, troches, lozenges, aqueous or oily suspensions, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavouring agents, colouring agents and preserving agents in order to provide a pharmaceutically elegant and palatable preparation.

Formulations for oral use include tablets which contain the active ingredients in admixture with non-toxic pharmaceutically acceptable excipients. These excipients may be, for example, inert diluents, such as calcium carbonate, sodium chloride, lactose, calcium phosphate or sodium phosphate; granulating and disintegrating agents, for example, potato starch, or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin, or olive oil.

Aqueous suspensions usually contain the active materials in admixture with appropriate excipients. Such excipients are suspending agents, for example, sodium carboxymethylcellulose, methylcellulose, hydroxypropylmethylcellulose, sodium alginate, polyvinylpyrrolidone, gum tragacanth and gum acacia; dispersing or wetting agents which may be a naturally-occurring phosphatide, for example, lecithin; a condensation product of an alkylene oxide with a fatty acid, for example, polyoxyethylene stearate; a condensation product of ethylene oxide with a long chain aliphatic alcohol, for example, heptadecaethyleneoxycetanol; a condensation product of ethylene oxide with a partial ester derived from fatty acids and a hexitol such as polyoxyethylene sorbitol monooleate; or a condensation product of ethylene oxide with a partial ester derived from fatty acids and hexitol anhydrides, for example, polyoxyethylene sorbitan monooleate. The aqueous suspensions may also contain one or more preservatives, for example, methyl, ethyl or n-propyl p-hydroxybenzoate; and one or more colouring agents; one or more flavouring agents; and one or more sweetening agents such as sucrose or saccharin.

Oily suspensions may be formulated by suspending the active ingredient in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents such as those set forth above, and flavouring agents may be added to provide a palatable oral preparation. These compositions may be preserved by the addition of an antioxidant such as ascorbic acid.

Dispersible powders and granules suitable for preparation of an aqueuos suspension by the addition of water provide the active ingredient in admixture with a dispersing or wetting agent, suspending agent and one or more preservatives. Suitable dispersing or wetting agents and suspending agents are exemplified by those already mentioned above. Additional exipients, for example, sweetening, flavouring and colouring agents, may also be present.

The pharmaceutical composition of the invention may also be in the form of oil-in-water emulsion. The oily

phase may be a vegetable oil, for example, olive oil or arachis oil, or a mineral oil, for example, liquid paraffin or mixtures of these. Suitable emulsifying agents may be naturally-occurring gums, for example gum acacia or gum tragacanth; naturally-occurring phosphatides, for example, soybean licithin; and esters including partial esters derived from fatty acids and hexitol anhydrides, for example, sorbitan mono-oleate, and condensation products of the said partial esters with ethylene oxide, for example, polyoxyethylene sorbitan monooleate. The emulsions may also contain sweetening and flavouring agents.

Syrups and elixirs and may be formulated with sweetening agents, for example glycerol, sorbitol or sucrose. Such formulations may also contain a demulcent, a preservative and flavouring and colouring agents.

Naturally, the therapeutic dosage range for the compounds of the present invention will vary with the size and needs of the host treated and the particular pain or disease symptom being treated. However, generally speaking, the following dosage guidelines will suffice. On parenteral local administration or oral administration of a compound of the present invention derived from a drug containing a carboxylic acid functional group, application of an amount of the high molecular weight prodrug corresponding to an amount of the parent drug of from 1 to 500 mg should suffice. In case of parenteral or oral administration of a prodrug of an antiinflammatory steroid or derived from other drugs containing a hydroxy functional group, application of a dose of from 0.1 to 200 mg, as calculated on basis of free steroid, should suffice.

From the foregoing description, one of ordinary skill in the art can easily ascertain the essential characteristics of the present invention and, without departing from the spirit and scope thereof, can make various changes and/or modifications of the invention to adapt it to various usages and conditions. As such, these changes and/or modifications are properly, equitably and intended to be within the full range of equivalence of the following claims.

*Preparation of the high molecular weight prodrugs of formula 1*

In formula 1, the moiety A - $(CH_2)_n$ - B - D, where A, n, B, D are as defined above, designates a ligand covalently attached to a polysaccharide/polysaccharide derivative. In the case where A is a carbonyl group the ligands of formula 1 represent acyl residues of various carboxylic acids comprising the following chemical structures:

$$R_1 - COOH \qquad (V)$$

wherein $R_1$ - CO are as defined above;

$$R_1 - CO - O - (CH_2)_n - COOH \qquad (X)$$

wherein $R_1$ - CO and n are as defined above;

$$R_1 - CO - NR - (CH_2)_n - COOH \qquad (Y)$$

wherein n, R and $R_1$ - CO are as defined above, and

$$R_2 - O - CO - (CH_2)_n - COOH \qquad (Z)$$

wherein n and $R_2$ - O are as defined above.

The carboxylic acid compounds given by the formulas V, X, Y and Z can be coupled to polysaccharides through ester linkages by a variety of synthetic routes to give the produgs of formula 1. Letting the term ligand-COOH represent any of the carboxylic acid structures given by the formulas V, X, Y and Z, a generally applicable process (method a) comprises reacting the carboxylic acid agent of formula E or a salt thereof

$$\text{ligand - COOH} \qquad (E)$$

wherein ligand-COOH is as defined above, with a polysaccharide/polysaccharide derivative during the formula F:

$$PS - OH \qquad (F)$$

wherein PS - O is as defined above in connection with formula 1. The reaction is conducted in the presence of a suitable dehydrating agent, for example N,N′-dicyclohexylcarbodiimide. The reaction utilizing an acid starting material is conveniently carried out in an inert solvent such as pyridine, formamide, dimethylsulphoxide, N,N-dimethylformamide containing 1-5% w/v LiCl and feasible mixtures hereof, at a temperature of from 0° to 60°C, for from 1 to 10 days. A catalyst such as 4-dimethylaminopyridine or p-toluenesulphonic acid may be added.

Another method (method b) for preparing compounds of this invention comprises reacting a compound of formula F with an acid chloride of formula G, derived from an acid of formula (E):

$$\text{ligand - COCl} \qquad (G)$$

In the reaction employing an acid chloride starting material, the process can be conveniently carried out by reacting the compounds of formula F with the desired acid chloride in an inert solvent such as formamide, pyridine, chloroform, dichloromethane, dimethylformamide or water, at room temperature to reflux, for from 1 to 24 h, in the presence of an acid scavenger such as an alkali metal carbonate, or an organic base such as triethylamine or pyridine.

The acid chlorides of formula G which can be used in the above method are prepared from the corresponding acids by known means, e.g. by treatment of the acid with thionyl chloride or oxalyl chloride. Instead of acid chlorides, acid anhydrides or mixed anhydrides may be used.

The starting materials of formula X are also prepared by known means, e.g. by treatment of the appropriate ω-hydroxycarboxylic acid with an acid chloride or acid anhydride of the parent carboxylic acid ($R_1$-COOH) as represented by the following chemical equation for an acid chloride:

$$R_1\text{-COCl} + HO\text{-}(CH_2)_n\text{-COOH} \rightarrow R_1\text{-COO-}(CH_2)_n\text{-COOH}$$

In addition, the acids of formula x are prepared from the parent acids (i.e. $R_1$ - COOH) by reacting the acid or a salt of the acid (e.g. a metal or triethylammonium salt) with compounds of formula H

$$W - (CH_2)_nCOOCH_2C_6H_5 \qquad (H)$$

wherein n is defined above and W is a suitable leaving group (e.g. halogen such as Cl, Br or I, or a methansulphonyloxy or toluenesulphonyloxy group) or with compounds of the formula J

$$W - (CH_2)_n - CONH_2 \qquad (J)$$

wherein W and n are as defined above. The intermediates obtained therefrom, i.e. $R_1$-COO-$(CH_2)_n$ $COOCH_2C_6H_5$ and $R_1$-COO$(CH_2)_n$-$CONH_2$, are subsequently transformed to the compounds of formula X by e.g. hydrogenation or acid hydrolysis. Several compounds of formula X and methods for preparing them are known from the literature, see e.g. Boltze et al. (1980) and Concilio and Bongini (1966).

The starting materials of formula Y are also prepared by known means, e.g. by treatment of the appropriate ω-aminocarboxylic acid with an acid chloride, an acid anhydride or a mixed anhydride of the parent carboxylic acid ($R_1$-COOH) as represented by the following chemical equation for an acid chloride:

$$R_1\text{-COCl} + H_2N\text{-}(CH_2)_n\text{-COOH} \rightarrow R_1\text{-CONH-}(CH_2)_n\text{-COOH}$$

Further, the acids of formula Y are prepared by aminolysis of activated esters (e.g. phenyl or N-hydroxysuccinimide esters) of the parent acids ($R_1$-COOH) effected by an appropriate ω-aminocarboxylic acid. Several methods for preparing compounds of the formula Y are reported in the literature concerning peptide synthesis.

The starting materials of formula Z are prepared according to known methods by reacting the alcoholic drug ($R_2$-OH) with a compound of formula K

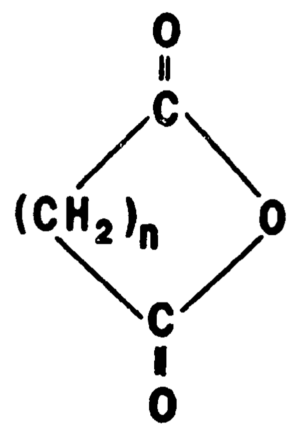

(K)

wherein n is 2 or 3, or by half-hydrolysis of intermediate diester compounds, i.e. $R_2OOC$-$(CH_2)_n$-$COOR_2$, obtained from the parent alcoholic drugs and an appropriate dicarboxylic acid by means of the various acylation methods described above. Several compounds of formula Z and methods for preparing them are known from the literature, see e.g. Vermeersch et al. (1985) and Yamamoto et al. (1971).

A third method (method c) for preparing compounds of the present invention comprises reacting a compound of the formula L or a salt thereof

$$PS - O - A - (CH_2)_n - COOH \qquad (L)$$

wherein PS-O, A and n are as defined above in connection with formula 1, with a compound having the formula M

$$R_2W \qquad (M)$$

wherein $R_2$ is the corresponding moiety in the group $R_2$-O defined above in connection with formula $1_2$ and W is a suitable leaving group (e.g. halogen such as Cl, Br or I, or a methansulphonyloxy or toluenesulphonyloxy group). The reaction is preferably carried out in an inert solvent (e.g. N,N-dimethylformamide, formamide, dimethylsulphoxide or water). An equivalent of an organic base such as triethylamine or tetramethylguanidine is typically added or crown ethers are used as phase-transfer catalysts. If W in formula M is chlorine catalytic amounts of an iodide salt may be added to the reaction mixture. The reaction is carried out at a temperature

of from room temperature to the boiling point of the solvent, and for a period of time of from 0.5 to 48 hours.

The starting materials of formula L, in which A is absent and n and PS-O are as defined above, are prepared by known means, e.g. by treating the polysaccharide (PS-OH) with a compound of formula N

$$W - (CH_2)_n - COOH \qquad (N)$$

wherein W and n are as defined above. Several compounds of formula N and methods for preparing them are known from the literature, see e.g. US Patent No 2,997,423.

The starting materials of formula L, in which A is a carbonyl group, n is 2 or 3 and PS-O is as defined above, are prepared by acylating the polysaccharide with a compound of formula K (Groff et al. (1982)).

Formula 1 encompasses further prodrug compounds of the carbonate ester type of formula O

$$PS - O - CO - O - R_2 \qquad (O)$$

wherein PS-O and $R_2$-O are as defined above. Thus, a fourth method (method d) for preparing compounds of the present invention comprises reacting a compound of the formula P

$$R_2 - O - COCl \qquad (P)$$

wherein $R_2$-O is as defined in connection with formula $1_2$ with a polysaccharide/polysaccharide derivative of formula F. The reaction is preferably carried out in the absence of water in a solvent such as pyridine or dimethylformamide, at a temperature of from 0° to 60°C, and for a period of time of from 1 to 48 hours. Theoretically, compounds of formula O might also be obtained by reacting the alcoholic drug ($R_2OH$) with a phosgene activated polysaccharide. The latter procedure, however, results in undesirably low yields and furthermore results in unpredictable crosslinking reactions between the polysaccharide carrier molecules themselves.

The starting material of formula P are prepared by known means, e.g. by treating the alcoholic drug compound ($R_2$-OH) with phosgene (see for example Havron et al. (1974)).

While the basic methods described above can be used to prepare any of the compounds of the invention, certain conditions and/or modifications therein are made in specific instances. Thus, for example, the basic methods may be modified in the cases where the desired product of formula 1 contains free aliphatic amino or hydroxy groups which, if present in the acid starting material, would undergo undesired side reaction and/or interfere with the desired course of the above-described ester information. In such cases, the compounds of formula F are reacted with an acid of the formula Q

$$R_P - COOH \qquad (Q)$$

wherein $R_P$-COO- is the amino or hydroxylprotected acyloxy residue of a carboxylic acid agent ($R_1$-COOH) containing amino or hydroxy groups. The amino or hydroxy function in the parent acids of the formula $R_1$-COOH are converted to their protected counterparts in formula Q by known methods, e.g. those known in the art of peptide synthesis. For example, amino groups are conveniently protected by the carbobenzoxycarbonyl or t-butyloxycarbonyl group. The compound of formula Q or its corresponding acid chloride is subsequently reacted with a compound of formula F, as described *supra*, to afford the compound corresponding to formula 1, but containing a protected acyloxy residue, i.e. $R_P$-COO- as defined above in place of $R_1$-COO- in formula $1_1$. That protected compound is then deprotected by known methods, e.g. by hydrogenation or hydrolysis. The above described process variations involving the addition and ultimate removal of protecting groups is only used when the free amino or hydroxy functions are in need of protection.

*Brief description of the drawings*

*Fig. 1* shows a gel permeation chromatogram of a ketoprofen-dextran T-10 conjugate with DS of 5.1 on Sephadex G-10 ($V_t$~150 ml). The fractions were analyzed by the anthrone method (o) and absorbance at 266 nm (Δ).

*Fig. 2* shows a calibration curve of log $M_w$ vs. retention volumes, $V_R$, for dextran standards chromatographed on an Ultrapac TSK G 3000 PW column.

*Fig. 3* shows a plot of the average molecular weight, $M_w$, for a dextran T-70-naproxen ester conjugate with DS of 6.9 (o) and the parent dextran (Δ) by LALLS.

*Fig. 4* shows the correlation between the hydrodynamic volume, [η], and the degree of substitution of dextran T-70-naproxen ester conjugate.

*Fig. 5* shows pH-rate profiles for hydrolysis of dextran T-70-NSAID ester conjugates at 37°C and the ionic strength = 0.5. Conjugates derived from ibuprofen (o), ketoprofen (Δ), fenoprofen (□), diclofenac (■) and naproxen (▲).

*Fig. 6* shows a plot of intact conjugate plasma concentration versus time for intravenous administration of a dextran T-40-naproxen ester conjugate (o) and a dextran T-70-naproxen ester conjugate (Δ) to one male rabbit (~3.5 kg) as determined by HP(SEC) chromatography.

*Fig. 7* shows plots of naproxen blood concentration versus time after oral administration of naproxen (o) and an equimolar amount of a dextran-naproxen conjugate ($M_w$ 71,000) (Δ) to pigs. The data are the average

from three pigs (weight approximately 40 kg).

*Fig. 8* shows plots of naproxen plasma concentration versus time after oral administration of naproxen (Δ) and an equimolar amount of a dextran-naproxen conjugate ($M_w$ 71,200) (o) to rabbits. The data are the average from two rabbits (weight approximately 3 kg).

*Fig. 9* shows plots of average naproxen plasma concentration versus time from 3 pigs after oral administration of solutions of dextran prodrugs varying in molecular size (all doses corresponding to 3.6 mg naproxen per kg body weight). (□): $M_w$ 500,000 (DS 6.8); (o): $M_w$ 70,000 (DS 8.2); (●): $M_w$ 40,000 (DS 6.9); (Δ) $M_w$ 10,000 (DS 7.1).

*Characterization of the conjugates*

Derivatives prepared as described above all had spectroscopic properties (IR and $^1H$ NMR) and elemental analysis (C, H, and N) in agreement with their structures. Due to the nature of the high molecular weight prodrugs of this invention, however, full characterization of the conjugates, besides determination of liberation kinetics of the parent active agent from the prodrug also includes determination of (a) the degree of substitution, (b) distribution of ligands along the polymer chains, (c) the molecular size distribution (e.g. the weight average molecular weight and the number average molecular weight), and (d) the hydrodynamic volume.

*a. The degree of substitution*

The degree of substitution was determined by hydrolysis of the individual polysaccharide prodrug. The released active parent drug (for example ibuprofen) was quantitated by reversed-phase HPLC. In general an accurately weighted amount of the individual conjugate corresponding to about 5 mg was dissolved in 25.00 ml of 0.1 N sodium hydroxide and the solution was heated to 60°C for 10 min in a water bath. After cooling 500 μl samples were withdrawn and added to 500 μl of 0.1 N hydrochloric acid. The resulting solutions were assayed for the parent drug compound by HPLC (the HPLC analytical procedures are described below in connection with the chemical kinetic studies). The absence of non-covalently linked parent drug in the conjugate was confirmed by dissolving a weighted amount of the polymer prodrug (~5 mg) in 25.00 ml 0.05 M phosphate buffer pH 6.0 and the solution was analyzed immediately for free parent drug. The degree of substitution (DS) has throughout this invention been expressed as the percentage of mg parent drug released per mg of the conjugate.

*b. Distribution of ligands*

The distribution of ligands along the polysaccharide chains was assessed by gel filtration. A 10 ml portion of the prodrug conjugate corresponding to 200 mg of the polysaccharide was applied to a Pharmacia column K 26/70, packed with Sephadex G-10 ($V_t$ = 150 ml), and eluted with distilled water. A flow rate of 1.9 ml $min^{-1}$ was maintained by use of a constant-speed peristaltic pump and the effluent was collected in 5 ml fractions. The fractions containing the substituted polysaccharides were analyzed by measuring the UV-absorbance at $\lambda_{max}$ for the individual liganded drug and by the anthrone reaction (according to the nordic pharmacopoeia) or by measurement of the optical rotation of the solutions. The latter two procedures are used in order to quantitate the pure polysaccharide content in the fractions. A representative example of the gelfiltration procedure is shown in Fig.1, where a ketoprofen-dextran T-70 conjugate with DS of 5.1 has been chromatographed. The shapes of the elution profiles as determined by UV-measurement at 266 nm and the anthrone reaction, respectively, are almost identical, strongly indicating that the ligands are distributed uniformly along the dextran chains.

*c. The average molecular weights*

In order to determine the polydispersity ($M_w/M_n$) of the high molecular weight prodrugs of this invention, the number average molecular weight ($M_n$) was determined by end-group analysis in accordance with the Somogyi phosphate methods (Isbell et al. (1953)). Measurements of the weight average molecular weight ($M_w$) of the prodrugs were performed by high-performance size exclusion chromatography (HP(SEC)) using a TSK G 3000PW column. This method is applicable to all polysaccharide prodrugs in which ligand fixation is accomplished through an ester bond. Prior to chromatography the conjugates were hydrolyzed to give the parent polysaccharide carrier (0.1 N sodium hydroxide for 10 min at 60°C). After neutralization the solutions were applied to the column and eluted with a mobile phase consisting of 0.05 M phosphate buffer pH 7.0 -acetonitrile (85:15 v/v). The column effluent was monitored employing a refractive index detector. For example in case of dextran

prodrugs the molecular weights of hydrolyzed conjugates were calculated from a standard curve (log $M_w$ *versus* $V_R$ in Fig. 2) based on well-defined dextran fractions (nordic pharmacopoeia standards) using the following expression:

$$\log M_w = 9.515 - 0.376 \times V_R$$

wherein $V_R$ refers to the retention volume of the parent dextran polymer obtained after hydrolysis. Hence, with the knowledge of the degree of substitution of the conjugate the molecular weight of the actual dextran prodrug was calculated. In order to check the validity of the above procedure $M_w$ of a conjugate was furthermore in a few cases obtained from low angle light scattering measurements (LALLS). Fig. 3 shows the results for a dextran-naproxen derivative with DS of 6.9 and for reasons of comparison a curve of a dextran T-70 sample is included. In LALLS-experiments the weight average molecular weight, $M_w$, can be calculated from the equation:

$$\frac{K \times C}{R_\theta} = \frac{1}{M_w}(1 + 2\Gamma_2 \times c)$$

where c is the concentration of the dissolved compound and $R_\theta$ is the excess Rayleigh scatter. $\Gamma_2$ refers to the second virial coefficient corresponding to the excluded volume. K is a constant defined by:

$$K = \frac{2\pi^2 n_o^2}{N_A \times \lambda^4}\left(\frac{dn}{dc}\right)^2$$

where $\pi$ is the osmotic pressure of the sample solution, $n_0$ and n are the refractive indicies of the solvent and the solution, respectively. $N_A$ and $\lambda$ symbolize the Avogadro number and the wavelength of the incident light, respectively. The intercept of the linear plot of K x c/ $R_\theta$ against c is equal to $M_w^{-1}$ and the slope is given by $2\Gamma_2/M_w$. By LALLS-measurement $M_w$ for the dextran-naproxen conjugate was determined to 71,500. By HP(SEC) analysis the $M_w$ of the conjugate was determined to 70,100. Thus the $M_w$ values obtained by the two methods agree within 2%, strongly suggesting that the HP(SEC) procedure is feasible for routine analysis.

*d. Hydrodynamic volume*

The viscosities of aqueous solutions of the parent polysaccharide carrier and the corresponding prodrugs were measured in an Ubbelohde viscometer at 20 ± 0.01°C. A representative example is given in Fig.4, showing the influence of the degree of substitution (DS) on the hydrodynamic volume of dextran-naproxen conjugates derived from a parent dextran with $M_w$ of 69,700, expressed by the limiting viscosity number, $[\eta]$. The correlation between the two parameters can be described as:

$$[\eta] = -2.45DS + 30.6$$

Table 1 comprises representative data for the conjugates including Huggins constant, k′, calculated from:

$$\eta_{spec}/c = [\eta] + k'[\eta]^2 \times c$$

where $\eta_{spec}$ is the specific viscosity and c is the solute concentration. As seen from Fig. 4, the limiting viscosity number descreases with increasing DS. Similar results are obtained for other non-charged conjugates of this invention, with the general observation that introduction of more lipophilic agents into the dextran backbone produces a more pronounced reduction of the hydrodynamic volume.

Table 1

Hydrodynamic volume (η), and Huggins constant, k', for dextran T-70-naproxen ester conjugates with varying degrees of substitution (DS) in aqueous solution (20°C).

| DS | (η) (ml/g) | k' |
|---|---|---|
| 0 | 29.1 | 0.40 |
| 2.1 | 25.7 | 0.54 |
| 3.9 | 22.1 | 0.86 |
| 6.9 | 13.0 | 1.39 |

The present invention is further illustrated by the following examples which, however, are not construed to be limiting. All values for degrees of substitution (DS) listed in the examples correspond to mg parent drug released per 100 mg of the conjugate.

EXAMPLE 1

*O-[(+)-6-Methoxy-α-methyl-2-naphthaleneacetyl]-dextran T-70*

Naproxen (1.0 g, 4.3 mmol) was dissolved in 20 ml of a mixture of formamide-pyridine (1:1) under stirring. The solution was cooled on ice and N,N'-dicyclohexylcarbodiimide (990 mg, 4.8 mmol) and 4-dimethylaminopyridine (54 mg, 0.44 mmol) were added. After 30 min a solution of dextran T-70 ($M_w$ = 65,000, $M_n$ = 34,600) (1.0 g, 0.014 mmol) in 20 ml of formamide-pyridine (1:1) was added. After stirring at room temperature for 4 days the mixture was diluted with 20 ml of distilled water containing 50 mg sodium chloride and after 10 min the reaction mixture was filtered. The conjugate was precipitated from the filtrate by addition of excess of ethanol. After standing overnight at 5°C the reaction mixture was decanted and the residue was dissolved in 10 ml of distilled water and desalted by gelfiltration by using a Pharmacia column K-26/70 packed with Sephadex G-10. The column was eluted with distilled water and the fractions containing the dextran prodrug were pooled and lyophilized, yielding 0.7 g (68% based on dextran) of the title compound. By employment of the aforementioned methods the conjugate was characterized by the following parameters: DS = 6.9; $M_w$ = 70,800; $M_w/M_n$ = 1.83; water-solubility > 25% w/v (25°C).

EXAMPLE 2

The compound in Example 1 with a DS of 1.2 was also prepared by the following procedure:

Naproxen (1.0 g; 4.3 mmol) was dissolved in 50 ml of a 5% w/v lithium chloride solution in N,N-dimethylformamide under stirring. The solution was cooled on ice and N,N'-dicyclohexylcarbodiimide (990 mg; 4.8 mmol) and p-toluenesulphonic acid (40 mg;0.23 mmol) were added. After 30 min a solution of dextran T-70 (1.0 g; 0.0014 mmol) in 50 ml of a 5% w/v lithium chloride solution in N,N-dimethylformamide was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol. After standing overnight at 5°C, the reaction mixture was decanted and the residue was dissolved in 10 ml of distilled water and desalted and lyophilized as described in Example 1, yielding 0.53 g (50%) of the title compound. DS = 1.2; $M_w$ = 70,300; $M_w/M_n$ = 1.86; water-solubility > 25% w/v (25°C).

EXAMPLE 3

The compound in Example 1 with a DS of 0.42 was further prepared by the following procedure:

Naproxen (1.0 g, 4.3 mmol), dextran T-70 (1.0 g, 0.014 mmol) and N,N'-dicyclohexylcarbodiimide (990 mg; 4.8 mmol) were dissolved in 40 ml of dimethylsulphoxide and left for two days at room temperature. Excess of acetone was added and the mixture was left overnight at 5°C. After decantation the precipitate was treated with 15 ml of distilled water and filtered. The filtrate was desalted by gelfiltration and lyophilized as described in Example 1, yielding 0.36 g (35%) of the title compound. DS = 0.42; $M_w$ = 70,100; $M_w/M_n$ = 1.91; water solubility > 30 % w/v (25°C).

EXAMPLES 4-15

By following the procedures of the foregoing examples and various combinations hereof several more dextran-naproxen conjugates, derived from the same dextran T-70 ($M_w$ = 65,000; $M_n$ = 34,600), with different degrees of substitution were prepared. The reaction conditions and the characterization parameters are summarized in Table 2.

**Table 2**. Coupling of naproxen to dextran T-70 ($M_w$ = 65,000, $M_n$ = 34,600) employing DCC as condensing agent. Influence of the ratio of the reactants, catalyst and solvent composition on the degree of substitution of the dextran conjugates of formula 1.

| Example | Dextran T-70 (mmol) | Naproxen (mmol) | DMAP[a] (mmol) | DCC[b] (mmol) | Solvent[c] (ml) | Reaction (time (days) | DS[d] | $M_w/M_n$[e] | Water solubility[f] % w/v (25°C) |
|---|---|---|---|---|---|---|---|---|---|
| 4 | 0.014 | 0.9 | - | 1.0 | FMA-Pyr (45-15) | 2 | 0.3 | 1.86 | > 30 |
| 5 | 0.014 | 0.9 | - | 1.0 | FMA-Pyr (15-35) | 2 | 0.7 | 1.85 | > 30 |
| 6 | 0.014 | 2.2 | 0.22 | 2.4 | 5% LiCl in DMF | 3 | 1.7 | 1.83 | > 30 |
| 7 | 0.014 | 2.2 | 0.22 | 2.4 | FMA-Pyr (25-15) | 2 | 4.0 | 1.88 | > 25 |
| 8 | 0.028 | 4.3 | 0.44 | 4.8 | FMA-Pyr (30-30) | 5 | 6.0 | 1.86 | > 25 |
| 9 | 0.028 | 8.7 | 0.88 | 9.6 | FMA-Pyr (50-30) | 5 | 0.4 | 1.92 | > 30 |
| 10 | 0.014 | 2.2 | 0.22 | 2.4 | FMA-Pyr (40-40) | 10 | 2.1 | 1.87 | > 25 |
| 11 | 0.014 | 2.2 | 0.22 | 2.4 | FMA-Pyr (30-30) | 4 | 3.9 | 1.84 | > 25 |
| 12 | 0.007 | 2.2 | 0.22 | 2.4 | FMA-Pyr (15-15) | 3 | 10.3 | 1.86 | > 20 |
| 13 | 0.007 | 3.3 | 0.66 | 3.6 | FMA-Pyr (15-15) | 4 | 15.7 | 1.84 | > 20 |
| 14 | 0.014 | 2.2 | 0.66 | 2.4 | DMSO (40) | 4 | 3.8 | 1.86 | > 25 |
| 15 | 0.014 | 2.2 | - | 2.4 | DMSO-Pyr (30-10) | 4 | 2.8 | 1.87 | > 25 |

a: dimethylaminopyridine

b: N, N' - dicyclohexylcarbodiimide

c: FMA = formamide: Pyr = pyridine; - DMF = N, N - dimethylformamide; DMSO = dimethylsulphoxide

d: DS = degree of substitution

e: $M_w/M_n$ = polydispersity of the conjugate, as determined by HP(SEC) and end group analysis

f: Due to the tendency of the conjugates to form gels in highly concentrated solutions no attempts were made to measure the saturation concentrations

EXAMPLE 16

The compound in Example 1 with a specifically desired DS within the range 0.5 - 15 was also prepared by partial hydrolysis of highly substituted dextran-naproxen conjugates derived from the same parent dextran T-70 sample as described by the following example:

The compound in Example 13 (1 g; DS = 15.7 and thus containing 0.68 mmol naproxen ester bonds) was dissolved in 10 ml of distilled water. 0.1 N sodium hydroxide (2.5 ml; 0.25 mmol) was added and after mixing

the solution was left for 2 hours at room temperature. The solution was adjusted to pH 6.5 by addition of an appropriate amount of 0.1 N hydrochloric acid. The reacting mixture was desalted and lyophilized as described in Example 1, yielding 0.85 g (85%) of the title compound. DS = 10.0; $M_w/M_n$ = 1.86; water solubility > 20% (25°C).

EXAMPLE 17

The compound in Example 1 was also prepared by the following procedure:

Naproxen anhydride (2.0 g; 4.5 mmol) and 4-dimethylaminopyridine (54 mg; 0.44 mmol) were added to 30 ml of an ice-cooled mixture of formamide-pyridine (6:4) containing dextran T-70 (1.0 g; 0.014 mmol) under stirring. The reaction mixture was left for 2 days. The dextran conjugate was precipitated by addition of excess of ethanolafter addition of 5 ml of distilled water containing 50 mg of sodium chloride. After standing overnight the reaction mixture was decanted and the residue was treated with 15 ml of distilled water, filtered, desalted and lyophilized as described in Example 1, yielding 0.46 g (43%) of the title compound. DS = 2.1; $M_w$ = 70,400; $M_w/M_n$ = 1.86; water-solubility > 25% w/v (25°C).

Naproxen anhydride was synthesized by adding N,N'-dicyclohexylcarbodiimide (1.01 g; 4.9 mmol) to 20 ml of tetrahydrofurane containing naproxen (2.3 g; 10 mmol) and the mixture was left overnight at ambient temperature. Hereafter 250 µl of glacial acetic acid was added under vigorous stirring and the mixture was immediately filtrated. The filtrate was evacuated in vacuo. The residue was recrystallized from ethanol-water, yielding 700 mg (32%) of the title compound. M.p. 116-118°C.

EXAMPLE 18

The compound in Example 1 was also prepared by the following procedure:

Dextran T-70 (1.0 g; 0.014 mmol) was dissolved in 100 ml of a 5% w/v lithium chloride solution in N,N-dimethylformamide. After addition of 1 ml of pyridine 5 ml of a solution of naproxen acid chloride (approximately 5 mmol) in acetone was added dropwise under stirring, and the mixture was left for 2 days. The conjugate was precipitated by addition of excess of ethanol and left overnight at 5°C. After decantation the residue was dissolved in 15 ml of distilled water, filtered, desalted and lyophilized as described in Example 1, yielding 0.56 g (53%) of the title compound. DS = 4.6; $M_w$ = 70,500; $M_w/M_n$ = 1.88; water-solubility > 25% (25°C).

Naproxen acid chloride was obtained by dissolving naproxen (4.6 g, 20 mmol) in 25 ml of methylene chloride. After addition of 5.8 ml of thionyl chloride the mixture was refluxed for 2 hours. After cooling the organic solvent was evaporated in vacuo. The residue was dissolved in 20 ml of toluene and evaporated in vacuo. The latter procedure was done trice. Finally, the residue was dissolved in 20 ml of acetone and used without further purification.

EXAMPLE 19

The compound in Example 1 was also prepared by the following procedure:

Dextran T-70 (1.0 g, 0.014 mmol) was dissolved in 10 ml of distilled water and 2 M potassium hydroxide (2.3 ml. 5 mmol) was added. After cooling to 0°C 5 ml of a solution of naproxen acid chloride (approximately 5 mmol) in chloroform was added over 2 hours under stirring maintaining the cooling. Hereafter the reaction mixture was left overnight under stirring. The phases were separated and to the aqueous phase excess of ethanol was added. The precipitate was allowed to settle and after decantation the residue was dissolved in 15 ml of distilled water, filtered, desalted and lyophilized as described in Example 1, yielding 0.75 g (71%) of the title compound. DS = 8.9; $M_w$ = 70,600; $M_w/M_n$ = 1.86; water solubility > 20% (25°C).

The naproxen acid chloride was prepared as descibed in Example 18.

EXAMPLES 20-30

By following the procedures of the foregoing examples several more dextran-naproxen ester prodrugs were prepared. The data of the parent dextran fractions and the characterization parameters of the synthesized dextran-naproxen conjugates are shown in Table 3.

Table 3. Compounds of formula 1 wherein naproxen has been linked through ester bonds to dextran fractions of various molecular sizes and polydispersities (see also Table 2).

| Example | Dextran[a] | Parent dextran | | Dextran conjugate | | | |
|---|---|---|---|---|---|---|---|
| | | $M_w$ [b] | $M_w/M_n$ [c] | DS[d] | $M_w$ [b] | $M_w/M_n$ [c] | Water solubility (25°C)[e] |
| 20 | T-2000 | $2x10^6$ | - | 4.2 | $2.1x10^6$ | - | > 15% w/v |
| 21 | T-500 | $4.9x10^5$ | 2.64 | 8.7 | $5.3x10^5$ | 2.53 | > 15% w/v |
| 22 | T-500 | $4.9x10^5$ | 2.64 | 2.1 | $5.0x10^5$ | 2.50 | > 15% w/v |
| 23 | T-250 | $2.7x10^5$ | 2.53 | 10.3 | $3.0x10^5$ | 2.53 | > 15% w/v |
| 24 | T-250 | $2.7x10^5$ | 2.53 | 4.2 | $2.8x10^5$ | 2.31 | > 15% w/v |
| 25 | T-150 | $1.5x10^5$ | - | 12.2 | $1.7x10^5$ | - | > 10% w/v |
| 26 | T-150 | $1.5x10^5$ | - | 5.0 | $1.6x10^5$ | - | > 15% w/v |
| 27 | T-40 | $4.1x10^4$ | 2.91 | 8.5 | $4.4x10^4$ | 3.00 | > 20% w/v |
| 28 | T-40 | $4.1x10^4$ | 2.91 | 4.6 | $4.3x10^4$ | 2.83 | > 20% w/v |
| 29 | T-20 | $2.0x10^4$ | 1.24 | 7.1 | $2.1x10^4$ | 1.20 | > 20% w/v |
| 30 | T-10 | $1.0x10^4$ | 2.10 | 6.8 | $1.1x10^4$ | 1.94 | > 20% w/v |

a: Dextran fractions obtained from Pharmacia Fine Products or Sigma

b: Weight average molecular weight as determined by HP(SEC) or LALLS

c: Polydispersity

d: Degree of substitution

e: Due to the tendency of the conjugates to form gels in highly concentrated solutions no attempts were made to measure the saturation concentrations.

EXAMPLE 31

*N-[(+)-6-Methoxy-α-methyl-2-naphthaleneacetyloxy]-succinimide*

Naproxen (2.0 g, 8.7 mmol) was dissolved in 60 ml of tetrahydrofurane under stirring. After cooling on ice N,N'-dicyclohexylcarbodiimide (2.0 g, 9.6 mmol) was added and stirring was continued for 20 min. N-hydroxysuccinimide (1.0 g, 8.7 mmol) was added and the mixture was stirred overnight. The reaction mixture was filtered and the filtrate was evacuated in vacuo. The residue was dissolved in hot ethyl acetate and upon cooling crystals precipitated, yielding 1.5 g (53%) of the title compound. M.p. 122 - 123°C.

EXAMPLE 32

*N-[(+)-6-Methoxy-α-methyl-2-naphthaleneacetyl]-2-aminoacetic acid*

Naproxen N-hydroxysuccinimide ester (Example 31) (3.0 g, 9.0 mmol) dissolved in 35 ml of tetrahydrofurane was added under stirring to 25 ml of distilled water containing glycine (0.66 g, 9.0 mmol) and sodium hydrogencarbonate (1.5 g, 18 mmol). The reaction mixture was stirred at room temperature for 2 days and filtered. The filtrate was reduced to approximately 25 ml in vacuo and concentrated hydrochloric acid was added dropwise until pH of the filtrate reached 1.3. The precipitate formed after standing overnight at 5°C was collected by filtration and recrystallized from hot methanol, yielding 1.8 g (70%) of the title compound. M.p. 127 - 129°C.

EXAMPLE 33

*N-[(+)-6-Methoxy-α-methyl-2-naphthaleneacetyl]-3-aminopropionic acid*

The amide was prepared from β-alanine and naproxen N-hydroxysuccinimide ester (prepared as described in Example 31) by the procedure described in Example 32. The crude product was recrystallized from methanol-water. M.p. 122 - 123°C.

EXAMPLE 34

*N-[(+)-6-Methoxy-α-methyl-2-naphthaleneacetyl]-5-aminovaleric acid*

The amide was prepared from 5-aminovaleric acid and naproxen N-hydroxysuccinimide ester by the procedure described in Example 32. The crude product was recrystalized from methanol-water. M.p. 136 - 138°C.

EXAMPLE 35

*N-[(+)-6-Methoxy-α-2-naphthaleneacetyl]-6-aminocaproic acid*

The amide was prepared from 6-aminocaproic acid and naproxen N-hydroxysuccinimide ester by the procedure described in Example 32. The crude product was recrystallized from methanol-water. M.p. 97 - 99°C.

EXAMPLE 36

*N-[(+)-6-Methoxy-α-methyl-2-naphthaleneacetyl]-8-aminooctanoic acid*

The amide was prepared from 8-aminooctanoic acid and naproxen N-hydroxysuccinimide ester by the procedure described in Example 32. The crude product was recrystallized from methanol-water. M.p. 96 - 98°C.

EXAMPLE 37

*2-[(+)-6-Methoxy-α-methyl-2-naphthaleneacetyloxy]-acetic acid*

Naproxen acid chloride, prepared as described in Example 18, (approximately 20 mmol) dissolved in 20 ml of acetone was added dropwise to 20 ml of a cooled solution (0°C) of pyridine containing glycolic acid (2.4 g, 32 mmol) under stirring and left overnight. The organic solvent was removed in vacuo and the residue was taken up in 40 ml of destilled water and pH was adjusted to 2.7 by addition of 4 N hydrochloric acid. The precipitate formed was filtered, washed with water and recrystallized from ethanol-water, yielding 3.8 g (66%) of the title compound. M.p. 123 - 125°C.

EXAMPLES 38-45

By following the procedure described in Example 1 several compounds of formula 1 wherein naproxen amides of ω-aminocarboxylic acids (e.g. the compounds prepared according to the Examples 32-36) and naproxen esters of ω-hydroxycarboxylic acids (e.g. the compound given in Example 37) have been linked through ester bonds to dextran fractions of various molecular size and polydispersities. The data of the parent dextran fractions and the characterization parameters of the synthesized spacer arm linked dextran-naproxen prodrugs are shown in Table 4.

Table 4. Compounds of formula 1 wherein naproxen amides of ω-aminocarboxylic acids (compounds given in Examples 32-36) and naproxen esters of ω-hydroxycarboxylic acids (compound in Example 37) have been linked through ester bonds to dextran fractions of various molecular sizes and polydispersities.

| Example | Parent dextran | | Compound in Example X (c) | Dextran conjugate | | | |
|---|---|---|---|---|---|---|---|
| | $M_w$ (a) | $M_w/M_n$ (b) | | DS (d) | $M_w$ (a) | $M_w/M_n$ (b) | Water-solubility (25°C) (e) |
| 38 | $6.5x10^4$ | 1.88 | 32 | 4.2 | $6.7x10^4$ | 1.93 | > 15% w/v |
| 39 | $6.5x10^4$ | 1.88 | 33 | 3.9 | $6.7x10^4$ | 1.90 | > 15% w/v |
| 40 | $6.5x10^4$ | 1.88 | 34 | 5.1 | $6.8x10^4$ | 1.75 | > 15% w/v |
| 41 | $6.5x10^4$ | 1.88 | 35 | 3.7 | $6.7x10^4$ | 1.88 | > 15% w/v |
| 42 | $6.5x10^4$ | 1.88 | 36 | 4.0 | $6.7x10^4$ | 1.80 | > 15% w/v |
| 43 | $6.5x10^4$ | 1.88 | 37 | 4.6 | $6.7x10^4$ | 1.86 | > 20% w/v |
| 44 | $1.0x10^4$ | 2.10 | 37 | 4.0 | $1.1x10^4$ | 2.06 | > 20% w/v |
| 45 | $4.9x10^5$ | 2.64 | 37 | 3.7 | $5.0x10^5$ | 2.34 | > 20% w/v |

a: Weight average molecular weight as determined by HP(SEC) or LALLS

b: Polydispersity

c: The compound in Example number X linked to dextran

d: Degree of substitution

e: Due to the tendency of the conjugates to form gels in highly concentrated solutions no attempts were made to measure the saturation concentrations.

EXAMPLE 46

*O-[3-(11β,17-dihydroxy-6α-methylpregna-1,4-diene-3,20-dione-21-oxycarbonyl)propionyl]-dextran T-70*

3-(11β,17-dihydroxy-6α-methylpregna-1,4-diene-3,20-dione-21-oxycarbonyl)propionic acid (hereinafter named methylprednisolone-21-monosuccinate) (2.04 g, 4.3 mmol) was dissolved in 20 ml of a mixture of formamide-pyridine (1:1) under stirring. The solution was cooled on ice and N,N'-dicyclohexylcarbodiimide (990 mg, 4.8 mmol) and 4-dimethylaminopyridine (54 mg, 0.44 mmol) was added. After 30 min a solution of dextran T-70 ($M_w$ = 65,000, $M_n$ =34,600) (1,0 g, 0.014 mmol) in 20 ml of formamide-pyridine (1:1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight at 5°C. The reaction mixture was decanted and the residue was dissolved in 10 ml of distilled wter, desalted and lyophilized as described in Example 1, yielding 0.75 g (73%) of the title compound. DS = 2.1; $M_w$ = 68,000; $M_w/M_n$ = 1.87; water-solubility > 20% w/v (25°C). Methylprednisolone-21-monosuccinate was isolated from Solu-Medrol (UPJOHN).

EXAMPLE 47

*O-[3-(11β,17-dihydroxy-6α-methylpregna-1,4-diene-3,20-dione-21-oxycarbonyl)propionyl]-dextran T-40*

The dextran ester prodrug was prepared from methylprednisolone-21-monosuccinate and dextran T-40 ($M_w$ = 41.000; $M_w/M_n$ = 2.91) by the procedure described in Example 32. DS = 2.7; $M_w$ = 42,300; $M_w/M_n$ = 2,73; water-solubility > 20% w/v (25°C).

EXAMPLE 48

*O-[3-(11β,17-dihydroxy-6α-methylpregna-1,4-diene-3,20-dione-21-oxycarbonyl)propionyl]-dextran T-500*

The dextran ester prodrug was prepared from methylprednisolone-21-monosuccinate and dextran T-500 ($M_w$ = 499,000; $M_w/M_n$ = 2.64) by the procedure described in Example 32. DS = 1.8, $M_w$ = 508,000; $M_w/M_n$ = 2.78; water solubility > 20% w/v (25°C).

EXAMPLE 49

*O-[3-(11β,17-dihydroxy-pregn-4-ene-3,20-dione-21-oxycarbonyl)propionyl]-dextran T-70*

3-(11β,17-dihydroxy-pregn-4-ene-3,20-dione-21-oxycarbonyl)propionic acid (hereinafter named hydrocortisone-21-monosuccinate) (1.99 g, 4.3 mmol) was dissolved in 20 ml of a mixture of formamide-pyridine (1:1) under stirring. The solution was cooled on ice and N,N'-dicyclohexylcarbodiimide (990 mg, 4,8 mmol) and 4-dimethylaminopyridine (54 mg, 0.44 mmol) were added. After 30 min a solution of dextran T-70 ($M_w$ = 65,000, $M_n$ = 34,600) (1.0 g, 0.014 mmol) in 20 ml of formamide-pyridine (1.1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight at 5°C. The reaction mixture was decanted and the residue was dissolved in 10 ml of distilled water, desalted and lyophilized as described in Example 1, yielding 0.7 g (68%) of the title compound. DS = 1.8; $M_w$ = 66,200; $M_w/M_n$ = 1.88; water-solubility > 20% w/v (25°C).
Hydrocortisone-21-monosuccinate was isolated from Solu-Cortef (UPJOHN).

EXAMPLE 50

*O-[2-(3-Benzoylphenyl)propionyl]-dextran T-70*

Ketoprofen (1.09 g, 4.3 mmol) was dissolved in 20 ml of a mixture of formamide-pyridine (1:1) under stirring. The solution was cooled on ice and N,N'-dicyclohexylcarbodiimide (990 mg, 4.8 mmol) and 4-dimethylaminopyridine were added. After 30 min a solution of dextran T-70 ($M_w$ = 65,000; $M_n$ = 34,600) ( 1.0 g, 0.014 mmol) in 20 ml of formamide-pyridine (1:1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight

at 5°C. The reaction mixture was decanted and the residue was dissolved in 10 ml of distilled water, desalted and lyophilized as described in Example 1, yielding 0.73 g (71%) of the title compound. DS = 5.1; $M_w$ = 68,000; $M_w/M_n$ = 1.86; water-solubility > 20% w/v (25°C).

EXAMPLE 51

*O-[2-(3-Benzoylphenyl)propionyl]-dextran T-10*

The dextran ester prodrug was prepared from ketoprofen and dextran T-10 ($M_w$ = 10,300; $M_w/M_n$ = 2.1) by the procedure described in Example 50. DS = 5.2; $M_w$ = 10,800; $M_w/M_n$ = 2.1; water-solubility > 20% w/v (25°C).

EXAMPLE 52

*O-[2-(3-Benzoylphenyl)propionyl]-dextran T-500*

The dextran ester prodrug was prepared from ketoprofen and dextran T-500 ($M_w$ = 488,000; $M_w/M_n$ = 2.64) by the procedure described in Example 50. DS = 4.7; $M_w$ = 501,400; $M_w/M_n$ = 2.6; water solubility > 20% w/v (25°C).

EXAMPLE 53

*O-[2-(4-Isobutylphenyl)propionyl]-dextran T-70*

Ibuprofen (0.89 g, 4,3 mmol) was dissolved in 20 ml of a mixture of formamide-pyridine (1:1) under stirring. The solution was cooled on ice and N,N′-dicyclohexylcarbodiimide (990 mg, 4.8 mmol) and 4-dimethylaminopyridine (54 mg, 0.44 mmol) were added. After 30 min a solution of dextran T-70 ($M_w$ = 65,000, $M_n$ = 34,600) (1.0 g, 0.014 mmol) in 20 ml of formamide-pyridine (1:1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight at 5°C. The reaction mixture was decanted and the residue was dissolved in 10 ml of distilled water, desalted and lyophilized as described in Example 1, yielding 0.70 g (68%) of the title compound. DS = 3.9; $M_w$ = 67,400; $M_w/M_n$ = 1.8; water-solubility > 20% w/v (25°C).

EXAMPLE 54

*O-[2-(4-Isobutylphenyl)propionyl]-dextran T-10*

The compound was prepared from ibuprofen and dextran T-10 ($M_w$ = 10,300, $M_w/M_n$ = 2.1) by the procedure described in Example 53. DS = 5.5; $M_w$ = 10,800; $M_w/M_n$ = 2.2; water-solubility > 20% w/v (25°C).

EXAMPLE 55

*O-[2-(4-Isobutylphenyl)propionyl]-dextran T-500*

The compound was prepared from ibuprofen and dextran T-500 ($M_w$ = 488,000, $M_w/M_n$ = 2.64) by the procedure described in Example 53. DS = 4.2; $M_w$ = 500,000; $M_w/M_n$ = 2.7; water-solubility > 20% w/v (25°C).

EXAMPLE 56

*O-[±)-2-(3-phenoxyphenyl)propionyl]-dextran T-70*

Fenoprofen calcium dihydrate (2.4 g, 4.3 mmol) was dissolved in 20 ml of a mixture of formamide-pyridine (1:1) under stirring. The solution was cooled on ice and N,N′-dicyclohexylcarbodiimide (990 mg, 0.44 mmol) were added. After 30 min a solution of dextran T-70 ($M_w$ = 65,000, $M_n$ = 34,600) (1.0 g, 0.014 mmol) in 20 ml of formamide-pyridine (1:1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight at 5°C. The reaction mixture was decanted and the residue was dissolved in 10 ml of distilled water, desalted and

lyophilized as described in Example 1, yielding 0.67 g (65%) of the title compound. DS = 8.2; $M_w$ = 70,100; $M_w/M_n$ = 1.9; water-solubility > 20% w/v (25°C).

EXAMPLE 57

*O-[(±)-2-(3-phenoxyphenyl)propionyl]-dextran T-10*

The prodrug compound was prepared from fenoprofen and dextran T-10 ($M_w$ = 10,300; $M_w/M_n$ = 2.1) by the procedure described in Example 56. DS = 7.4; $M_w$ = 11,100; $M_w/M_n$ = 2.2; water-solubility > 20% w/v (25°C).

EXAMPLE 58

*O-[(±)-2-(3-phenoxyphenyl)propionyl]-dextran T-500*

The prodrug compound was prepared from fenoprofen and dextran T-500 ($M_w$ = 488,000, $M_w/M_n$ = 2.64) by the procedure described in Example 56. DS = 4.1; $M_w$ = 500,000; $M_w/M_n$ = 2.7; water-solubility > 20% w/v (25°C).

EXAMPLE 59

*O-[[2-(2,6-dichloroanilino)phenyl]acetyl]-dextran T-70*

Diclofenac sodium (1.4 g, 4.3 mmol) was dissolved in 20 ml of a mixture of formamide-pyridine (1:1) under stirring. The solution was cooled on ice and N,N'-dicyclohexylcarbodiimide (990 mg, 4.8 mmol) and 4-dimethylaminopyridine ( 54 mg, 0.44 mmol) were added. After 30 min a solution of dextran T-70 ($M_w$ = 65,000, $M_n$ = 34,600) (1.0 g, 0.014 mmol) in 20 ml of formamide-pyridine (1:1) was added. After stirring at room temperature for 4 days the mixture was diluted with 20 ml of distilled water containing 50 mg of sodium chloride and after 10 min the reaction mixture was filtered. The conjugate was precipitated from the filtrate by addition of excess of ethanol and left overnight at 5°C. The reaction mixture was decanted and the residue was dissolved in 10 ml of distilled water, desalted and lyophilized as described in Example 1, yielding 0.75 g (73%) of the title compound. DS = 1.1; $M_w$ = 65,400; $M_w/M_n$ = 1.8; water-solubility > 20% w/v (25°C).

EXAMPLE 60

*O-[[2-(2,6-dichloroanilino)phenyl]acetyl]-dextran T-10*

The compound was prepared from diclofenac sodium and dextran T-10 ($M_w$ = 10,300, $M_w/M_n$ = 2.1) by the procedure described in Example 59. DS = 1.0; $M_w$ = 10,400; $M_w/M_n$ = 2.1; water-solubility > 20% w/v (25°C).

EXAMPLE 61

*O-[[2-(2,6-dichloroanilino)phenyl]acetyl]-dextran T-500*

The compound was prepared from diclofenac sodium and dextran T-500 ($M_w$ = 488,000, $M_w/M_n$ = 2.64) by the procedure described in Example 59. DS = 1.0; $M_w$ = 492,800; $M_w/M_n$ = 2.7; water-solubility > 20% w/v (25°C).

*In vitro cleavage of dextran ester prodrugs*

*Reaction conditions.*

Release kinetics of the parent drug compounds from the corresponding dextran prodrugs of this invention was carried out in aqueous buffer solution over the pH range 4.5 - 10.0 at 37°C (and in order to determine the activation energies, at pH 4.5, from Arrhenius type plots at 50, 60, 65 and 70°C). Stability studies of the conjugates was further performed in human and rabbit plasma, in pig and rabbit liver homogenate, and in human synovial fluid derived from inflamed joints. The 1. order rate constants were derived from the slope of linear plots of $\log(B_\infty - B_t)$ *versus* time and for the slow reactions by employing the initial rate method (e.g. $(dB_t/dt)_i = k_{obs}A_o$), where $B_\infty$ and $B_t$ refer to the concentrations of regenerated parent drug at infinity and time t, respectively. $(dB_t/dt)_i$ represents the initial rate of parent drug formation, whereas $k_{obs}$ and $A_o$ are the pseudo-first-order rate constant and the initial dextran prodrug concentration, respectively. For the fast reactions the initial concentration of the conjugates was in the range 5 - 25 mg/10 ml and for the slow reactions in the range 50 - 250 mg/10 ml. At various times an aliquot of the solutions was withdrawn and analyzed by HPLC for liberated free drug and in some cases for intact dextran prodrug. In case of the biological reaction media the aliquot withdrawn was deproteinized with methanol, acetonitrile or 10 % trichloroacetic acid.

*Analytical methods.*

A HPLC method was used for the determination of the generated free NSAIDs (the HPLC procedure was further used for determination of the degree of substitution of the polysaccharide prodrugs). In this method a column (125 x 4.6 mm), packed with Spherisorb ODS 1 (5 μm particles), was eluted with a mobile phase consisting of methanol - 0.05 M phosphate buffer pH 7.0 (1-1 v/v). The flow rate was 1.0 ml/min and the column effluent was monitored at 266 nm. In this system the capacity factors of ibuprofen, ketoprofen, fenoprofen, diclofenac and naproxen were 2.8, 1.2, 1.8, 2.6 and 0.7, respectively. Quantitation of the compounds was done by measurements of peak heights in relation to those of standards chromatographed under identical conditions.

A HP(SEC) method (High-performance size exclusion chromatography) was used for the determination of the intact dextran prodrugs and the blood concentrations of dextran-naproxen ester prodrugs (compounds in Examples 7 and 28) after intravenous administration in rabbits. In this method a column, (250 x 8 mm), packed with Nucleosil Diol 7-OH (7 μm particles), was eluted with a mobile phase consisting of 0.05 M phosphoric acid - acetonitrile (90-10 v/v) at a flow rate of 1.0 ml/min. In case of dextran-naproxen conjugates the column effluent was monitored by fluorescens detection ($\lambda_{ex}$ 330 nm, $\lambda_{em}$ 360 nm). The other dextran conjugates were monitored spectrophotometrically in the UV range at an appropriate wavelength. In this system the retention times of conjugates (DS < 6.5%) derived from the employed parent dextran fractions are: Dextran T-10: 7.84; dextran T-20: 6.60; dextran T-40: 5.74; dextran T-70: 5.09; dextran 150: 5.05; dextran T-250: 5.05. Quantitation of the compounds was done by measurements of the areas in relation to those of standards chromatographed under the same conditions.

In Table 5 are presented the first-order rate constants for degradation of the various dextran T-70-NSAID ester prodrugs in aqueous solution over the pH range 4.48 - 10.0 (37°C and $\mu$ = 0.5) and in Fig. 5 the individual pH-rate profiles are shown. From the latter profiles it appears that the various dextran conjugates exhibit maximum stability in the pH range 4-5. In Table 6 are presented values of the activation energy, $E_a$, for hydrolysis of various dextran T-70-NSAID ester prodrugs at pH 4.48, calculated from the slopes of Arrhenius type plots, the equations of which are further included in Table 6. The latter equations have been used to calculate the stability (as represented by t(10%)), of the conjugates in aqueous solution at pH 4.48 at relevant storage temperatures (Table 7).

Table 5

Pseudo-first-order rate constants, $k_{obs}$, for degradation of dextran-NSAID ester prodrugs in aqueous solution in the pH range 1.14 - 10.00 at 37°C and an ionic strength of 0.5

| pH | $k_{obs}$ ($h^{-1}$) | | | | |
|---|---|---|---|---|---|
| | Dex-Ibuprofen (a) (T-70, DS = 3,9) | Dex-Ketoprofen (b) (T-10, DS = 5.2) | Dex-Fenoprofen (c) (T-10, DS = 7.4) | Dex-Diclofenac (d) (T-10, DS = 1.1) | Dex-Naproxen (e) (T-70, DS = 5.6) |
| 10.00 | $8.99 \times 10^{-1}$ | 2.80 | 1.67 | - | - |
| 9.54 | - | - | - | 3.01 | - |
| 9.05 | $1.40 \times 10^{-1}$ | $3.42 \times 10^{-1}$ | $2.02 \times 10^{-1}$ | $7.46 \times 10^{-1}$ | $1.64 \times 10^{-1}$ |
| 7.40 | $4.83 \times 10^{-3}$ | $7.43 \times 10^{-3}$ | $5.34 \times 10^{-3}$ | $1.78 \times 10^{-2}$ | $3.96 \times 10^{-3}$ |
| 6.54 | $6.59 \times 10^{-4}$ | $1.42 \times 10^{-3}$ | $1.00 \times 10^{-3}$ | $3.73 \times 10^{-3}$ | $4.34 \times 10^{-4}$ |
| 5.54 | - | $1.56 \times 10^{-4}$ | $8.81 \times 10^{-5}$ | $3.27 \times 10^{-4}$ | $4.44 \times 10^{-5}$ |
| 4.48 | $3.50 \times 10^{-5}$ | $4.08 \times 10^{-5}$ | $2.18 \times 10^{-5}$ | $1.18 \times 10^{-4}$ | $1.26 \times 10^{-5}$ |
| 1.14 | $5.33 \times 10^{-3}$ | $7.90 \times 10^{-3}$ | $3.64 \times 10^{-3}$ | - | $3.61 \times 10^{-3}$ |

(a): Compound in Example 53
(b): - - 50
(c): - - 56
(d): - - 59
(e): - - 7

**Table 6.** Values of the activation energy ($E_a$) for hydrolysis of various dextran T-70-NSAID ester prodrugs at pH 4.48 and the corresponding Arrhenius equations.

| Conjugate (a) | $E_a$ (kJ/mol) | Arrhenius equation (b) |
|---|---|---|
| Dex-Ibuprofen | 101.0 | $\ln k_{obs} = 28.9 - 12{,}140/T$ (n = 5, r = 0.97) |
| Dex-Ketoprofen | 94.9 | $\ln k_{obs} = 26.7 - 11{,}410/T$ (n = 5, r = 0.99) |
| Dex-Fenoprofen | 106.3 | $\ln k_{obs} = 30.6 - 12{,}790/T$ (n = 5, r = 0.99) |
| Dex-Diclofenac | 120.2 | $\ln k_{obs} = 37.6 - 14{,}450/T$ (n = 5, r = 0.99) |
| Dex-Naproxen | 88.5 | $\ln k_{obs} = 23.0 - 10{,}640/T$ (n = 5, r = 0.98) |

(a) : The dextran-NSAID compounds are those defined in Table 5

(b) : The dimension of the first-order rate, $k_{obs}$, is $hours^{-1}$ and T represents degrees Kelvin.

**Table 7. t(10%) for hydrolysis of various dextran T-70-NSAID ester prodrugs at 25°C and 5°C at pH 4.48.**

| Compound (a) | t(10%) (years) (b) | |
|---|---|---|
| | 25°C | 5°C |
| Dex-Ibuprofen | 1.68 | 31.5 |
| Dex-Ketoprofen | 1.31 | 20.6 |
| Dex-Fenoprofen | 2.72 | 59.7 |
| Dex-Diclofenac | 0.65 | 21.3 |
| Dex-Naproxen | 4.02 | 52.4 |

(a): The dextran-NSAID compounds are those defined in Table 5

(b): t(10%) is the time for 10% degradation of the conjugates.

As seen from Table 8 neither the molecular weight nor the degree of substitution effects the stability of conjugates containing the same liganded drug in aqueous solution. The liberation rates of the NSAID compounds from the corresponding dextran-NSAID ester prodrugs after incubation in an array of biological media are presented in Table 9.

**Table 8.** Half-lives t(50%) for hydrolysis of dextran-NSAID ester prodrugs with varying molecular weight and degree of substitution (DS) in 0.05 M phosphate buffer pH 7.40 at 37°C.

| Conjugate | Compound in Example | $M_w$ (parent dextran | DS | t(50%) at pH 7.40 and 37°C (hours) |
|---|---|---|---|---|
| Dex-Naproxen | 1 | 65,000 | 6.9 | 179 |
| Dex-Naproxen | 2 | 65,000 | 1.2 | 177 |
| Dex-Naproxen | 12 | 65,000 | 10.3 | 175 |
| Dex-Naproxen | 13 | 65,000 | 15.7 | 177 |
| Dex-Naproxen | 21 | 490,000 | 8.7 | 175 |
| Dex-Naproxen | 24 | 270,000 | 4.2 | 176 |
| Dex-Naproxen | 27 | 41,000 | 8.5 | 177 |
| Dex-Naproxen | 29 | 20,300 | 7.1 | 173 |
| Dex-Naproxen | 30 | 10,300 | 6.8 | 174 |
| Dex-Ketoprofen | 50 | 65,000 | 5.1 | 93 |
| Dex-Ketoprofen | 51 | 10,300 | 5.2 | 93 |
| Dex-Ketoprofen | 52 | 490,000 | 4.7 | 91 |
| Dex-Ibuprofen | 53 | 65,000 | 3.9 | 144 |
| Dex-Ibuprofen | 54 | 10,000 | 5.5 | 140 |
| Dex-Ibuprofen | 55 | 490,000 | 4.2 | 141 |
| Dex-Fenoprofen | 56 | 65,000 | 8.2 | 130 |
| Dex-Fenoprofen | 57 | 10,000 | 7.4 | 128 |
| Dex-Fenoprofen | 58 | 490,000 | 4.1 | 131 |

Table 9

Regeneration Half-lives (Hours) of NSAID compounds from Their Corresponding Dextran Ester Prodrugs in Different Biological Media (pH 7.40 and 37°C)

| NSAID | Dextran[a] | DS[b] | 5% Liver Homogenate | | 20% Human Synovial Fluid | 80% Plasma | | | Buffer pH 7.40 |
|---|---|---|---|---|---|---|---|---|---|
| | | | pig | rabbit | | Pig | Human | Rabbit | |
| Naproxen | T-70 | 5.6 | n.d. | 133 | 211 | 178 | 172 | 176 | 175 |
| Naproxen | T-10 | 9.9 | 128 | 126 | 194 | 161 | 134 | 126 | 179 |
| Naproxen | T-500 | 6.6 | 183 | 191 | 217 | n.d. | 169 | n.d. | 183 |
| Ketoprofen | T-10 | 5.2 | 88 | 102 | 120 | 203 | 74 | 89 | 93 |
| Fenoprofen | T-10 | 7.4 | 66 | n.d. | n.d. | 94 | 115 | 98 | 130 |
| Ibuprofen | T-70 | 3.9 | 77 | n.d. | n.d. | 159 | 123 | n.d. | 143 |
| Diclofenac | T-10 | 1.1 | 36 | n.d. | n.d. | n.d. | 29 | n.d. | 39 |

[a]: The dextran T-fractions refer to the Pharmacia samples.
[b]: DS = Degree of substitution
n.d. = not determined

Except from the dextran-ibuprofen conjugate the stability of the dextran-NSAID conjugates in the different

biological media are of the same order of magnitude as determined in aqueous buffer pH 7.40, strongly suggesting that the regeneration rates of the NSAID compounds from the conjugates proceed without enzyme facilitated hydrolysis. Regarding intra-articular administration it is of interest to note that the conjugates under investigation apparently are more stable in 20 % human synovial fluid (pH 7.40) than in aqueous buffer pH 7.40. In the synovial fluid the half-lives of the dextran conjugates derived from naproxen and ketoprofen are 210 and 120 hours, respectively. Assuming that the pH of the synovial fluid is lower than 7.40, due to increased metabolic activity in an inflamed joint, the conjugates may show even more pronounced sustained release properties.

*Bioavailability study*

The dextran-naproxen ester prodrugs described in Example 1 and 28, respectively, were administered parenterally to male rabbits (~3.5 kg). The administration of the compounds (1 ml of a 10 % w/v aqueous solution of the individual conjugate) was made intravenously into an auricular vein, whereas blood samples were taken by vein puncture of the other ear. After drug administration, blood samples were taken at appropriate intervals and the plasma fractions were assayed for intact dextran ester prodrug using the aforementioned fluorescens HP(SEC) method.

From the plasma concentration versus time profiles plasma half-lives for the T-40 and the T-70 dextran-naproxen ester conjugates of 58 and 90 min, respectively, were calculated (Fig.6). By extrapolation of the linear log plasma conc. versus time plots to time zero the initial plasma concentrations of the conjugates were compared to the corresponding theoretical values derived assuming that the total rabbit blood volumen amounts to 6 - 7 % of the total body weight. Although the amounts of conjugates excreted in the urine have not been measured the observed fine agreement between the experimentally and theoretically determined values of the initial plasma concentration indicates, that the conjugates are eliminated freely through the kidneys after intravenous administration without significant accumulation in the liver and other phagocytically active tissues. Thus, leaching to the systemic circulation of lower molecular weight fractions of dextran-NSAID conjugates from a local administration site (for example from a joint cavity) may be expected to result in a transient low plasma concentration due to the high ultrafiltration capacity of the kidneys.

To one rabbit a dose corresponding to 30 mg per kg body weight of naproxen ester conjugates derived from dextran T-40, T-70 and T-500, respectively, was administered intravenously successively with intervals of 1 week. After 3 weeks rest one further dose of the T-500 conjugate was given i.v.. The rabbit showed no apparent discomfort to this repeated administration.

Naproxen-dextran conjugates were administered orally to pigs. The absorption of the parent drug from the gastrointestinal tract was determined by measuring the naproxen concentration in plasma as function of time using the HPLC-procedure described above. The area under the curve was calculated from the equation:

$$AUC_{0-\infty} = AUC_{0-t} + c_t/\beta$$

where $\beta$ is the apparent elimination rate constant. The relative bioavailability (F%) of the dextran-naproxen conjugates was determined as the percentage of $AUC_{0-\infty}$ for the prodrugs in relation to that of free naproxen administered under identical conditions. In Fig. 7 the naproxen blood concentration-time profiles after oral administration of parent naproxen and a dextran-naproxen conjugate ($M_w$ = 71,000) are shown. The presented data are each the average obtained from three pigs (weight approximately 40 kg). A relative bioavailability of above 90% was calculated. Similar results were found by administering conjugates with molecular weight of 11,000, 40,000 and 505,000, respectively, see table 10 below.

**Table** 10 . Bioavailability of Naproxen after Oral Administration of Dextran-Naproxen Ester Prodrugs in Pigs[a]. Average Pharmacokinetic Parameters, Determined after Administrering Solutions of Dextran Prodrugs varying in Weight Average Molecular Weight, in Comparison to those obtained after p.o. and i.v. Administration of Solutions of Parent Naproxen.

| COMPOUNDS[b] | $C_{max}$ ($\mu g ml^{-1}$) | $T_{max}$ (h) | $\beta$ ($h^{-1}$) | AUC ($\mu g ml^{-1} h$) | AUC(p.o.) x 100 / AUC(i.v.) | AUC(conjugate)x100 / AUC(naproxen p.o.) |
|---|---|---|---|---|---|---|
| NAPROXEN i.v.[c] | 35 | - | 0.048 | 479 | - | - |
| NAPROXEN p.o.[c] | 19.7 | 2 | 0.049 | 439 | 91.6 | - |
| DEX-T-10 conjugate p.o. | 15.6 | 14 | 0.052 | 476 | 99.4 | 108.4 |
| DEX-T-40 conjugate p.o.[d] | 15.5 | 13 | 0.048 | 508 | 106.1 | 115.7 |
| DEX-T-70 conjugate p.o.[c] | 11.1 | 16 | 0.045 | 398 | 83.1 | 90.7 |
| DEX-T-500 conjugate p.o. | 11.6 | 18 | 0.044 | 422 | 88.1 | 96.1 |

[a] Each conjugate was administered to three pigs ranging in weight from 33 to 45 kg.
[b] Equal doses corresponding to 3.6 mg naproxen equivalents per body weight were given.
[c] From a previous study (8).
[d] A new group of three pigs were used.

The pigs were fasted for 18 hours prior to drug administration whereafter a dose corresponding to 3.6 mg naproxen per kg (or an amount of the conjugates corresponding to 3.6 mg free naproxen per kg) was given in the food. Throughout the experiment the pigs had free access to water and were fed while taking the blood samples. As seen from Fig.7 administration of dextran-naproxen prodrugs results in a delayed and a prolonged constant concentration of naproxen in plasma compared to administration of free drug. The characteristic lag-time of naproxen absorption after giving the conjugates orally has also been shown in rabbits (Fig. 8).

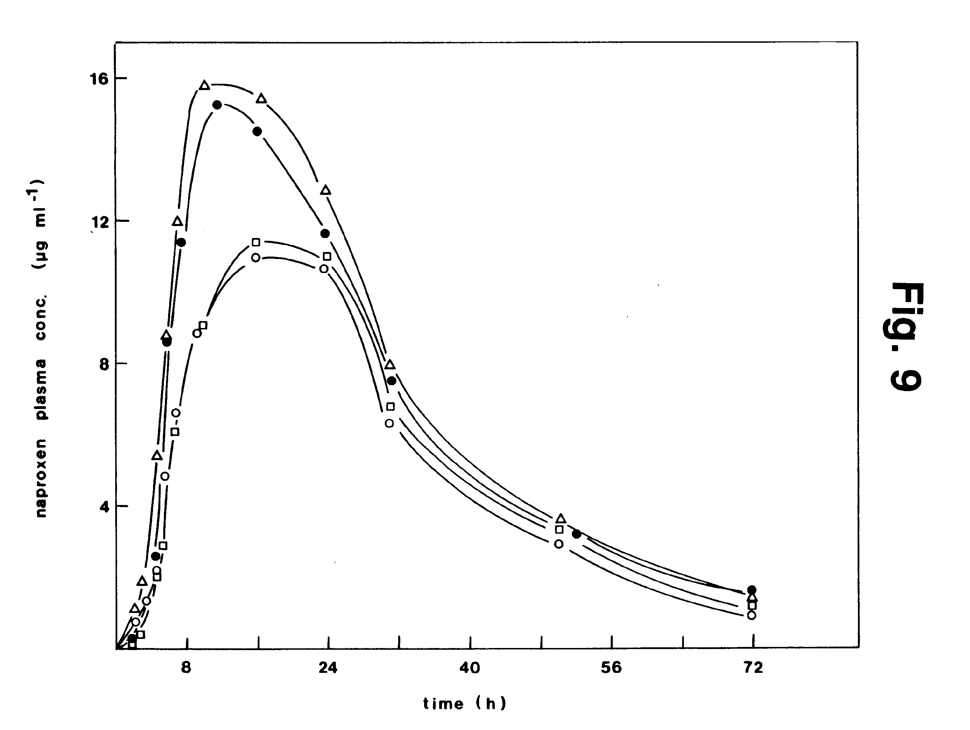

Similar absorption profiles were observed after giving conjugates of various molecular weights to pigs (Fig. 9).

In order to determine the region in the GI-tract in which naproxen was released from the conjugates, experiments were carried out in which conjugates were incubated in homogenates of various segments of the GI-tract with their contents, the segments being taken from rabbits and pigs, respectively.

Preparation of GI-tract homogenates

A male albino rabbit and a female pig (Danish landrace/Yorkshire) weighing approximately 3 and 45 kg, respectively, were used in this study. The animals were given standard diets before they were killed. About 30 min were required to excise the various parts of the gastrointestinal tract from each animal. The GI tract segments with their content were cut into small pieces, weighed, pooled in glass vials and stored at -20°C. After thawing the tissue homogenates were prepared by suspending each GI tract segment in twice the volume of cold 0.9% sodium chloride. The mixture was homogenized and centrifuged at 5,000 x g in a refrigerated (4°C) centrifuge. The resulting supernatant was frozen immediately in 2 ml portions. Due to the length of the pig colon (close to 2 m) homogenates of the proximal (colon I) and the distal part (colon II) were prepared separately. Colon samples without content were obtained after rinsing carefully the tissue with 0.9% sodium chloride.

**Kinetic measurements**

The reaction solutions were kept at 37±0.2°C in a constant-temperature water bath. The initial velocities of naproxen formation were in most cases monitored after adding 1000 μl of a 0.2 M phosphate buffer pH 7.40 containing 40±4 mg of the individual dextran conjugate to 1000 μl homogenate solution preheated to the temperature of study. 200 μl aliquots were withdrawn and deproteinized with 600 μl of methanol. The mixtures were vortexed and centrifuged at 10,000 x g for 4 min. During the sampling period, ranging from 6 to 60 min, six to eight samples were taken at suitable intervals. The methanolic supernatants were analyzed for liberated drug by using HPLC:

A column, 125 x 4.6 mm, packed with Spherisorb ODS-1 (5μm particles) was eluted with a mobile phase consisting of methanol - 0.02 M phosphate buffer pH 2.5 (65:35 v/v). The flow rate was maintained at 1.0 ml $min^{-1}$ and the column effluent was monitored at 271 nm. The HPLC apparatus was composed for a Hitachi L-6200 Intelligent pump, a Hitachi L-4000 variable wavelength detector, a Reodyne Model 7125 injection valve, a Hitachi 655A-40 auto sampler and a Hitachi D2000 chromato-integrator.Quantitation of the drug compound was done from peak area measurements in relation to those of external standards chromatographed under the same conditions.

The results are shown in tables 11 and 12 which demonstrate that the release of naproxen proceeds much faster in the caecum and the colon homogenates compared to homogenates from the upper sections of the GI-tract and that the molecular weight has only a minor effect on the liberation rates in the caecum and colon homogenates.

The degradation of the dextran naproxen ester conjugates in colon homogenates was also followed employing a high-performance size exclusion chromatography procedure (HP(SEC)). The chromatographic system consisted of a Hitachi Model 655A-11 solvent delivery pump, equipped with a variable wavelength Hitachi F1000 fluorescence detector, a Rheodyne Model 7125 injection valve with a 20μl loop and a Hitachi Model D2000 chromato-integrator. The column, 250 x 8 mm, was packed with spherically shaped Nucleosil Diol 7-OH particles (7μm) (Mackerey-Nagel, F.R.G.). During chromatography, the column was protected by a small pre-column packed with nucleosil Diol and by a silica saturation column positioned between the pump and the injection valve. The latter column was packed with LiChroprep SI 60, 15-25 μm (Merck, F.R.G.). The mobile phase was a mixture of acetonitrile - 0.05 M phosphoric acid (30:70 v/v). The flow rate was set at 1 ml $min^{-1}$ and the column effluent was monitored at exitation and emission wavelength of 330 and 360 nm, respectively. In addition to the detection of free as well as conjugated naproxen the HP(SEC) method was used to estimate the weight average molecular weights of the dextran conjugates as a function of time in the GI-tract homogenates.

For the HP(SEC) experiments a stock solution of a dextran T-70 naproxen ester prodrug in 0.2 M phosphate

buffer pH 7.40 was prepared (3.3 mg $ml^{-1}$). The reactions were initiated by adding 1000 µl of the stock solution to 2000 µl of the pure colon I homogenate or to equal volumes of the homogenate containing 50 mg glucose, 5 mg, 10 mg or 50 mg parent dextran T-70. At appropriate intervals 200 µl samples were taken and added to 400 µl of a 20% w/v trichloroacetic acid solution. After vortexing, the mixtures were centrifuged at 10,000 x g for 4 min. Due to the limited stability of the dextran ester conjugates in the precipitation solution HP(SEC), analysis was carried out immediately after the sample preparation.

From these experiments, it was found that the dextran chains were degraded while the naproxen moieties were still attached thereto whereby the liberation of naproxen did not take place until after at least partial degradation of the dextran polymer.

In summary, the experiments carried out on GI-tract sections demonstrated that naproxen is almost exclusively liberated in the caecum and colon and that the liberation in these sections involves initial degradation of the dextran polymer followed by release of naproxen from the smaller dextran polymer fragments resulting from the degradation. Thus, the dextran polymer is substrate for a depolymerase whereas the smaller polymer fragments are substrates for various hydrolases.

**Table 11 Initial Velocities of Naproxen Formation ($V_i$) after Incubation of a Dextran T-70-Naproxen Ester Prodrug with DS 8.3 in Homogenates Prepared from Various Segments of the GI Tract of Rabbits and Pigs with their content (37°C).**

| Homogenate/Buffer[a] | Rabbit | Pig | | |
|---|---|---|---|---|
| | $v_i$ (µg $ml^{-1}$/h) | $v_i$ (µg $ml^{-1}$/h) | pH (start) | pH (end) |
| Stomach | - | 11.2 | 7.3 | 7.5 |
| Duodenum | 7.1 | 7.4 | 7.4 | 7.4 |
| Jejunum | 6.0 | 7.1 | 7.4 | 7.4 |
| Ileum | 6.3 | 8.1 | 7.3 | 7.5 |
| Caecum | 65.1 | 86.5 | 7.4 | 7.5 |
| Colon I | 32.6 | 107.0 | 7.4 | 7.4 |
| Colon II | - | 111.2 | 7.4 | 7.4 |
| 0.1M Phosphate pH 7.40 | 6.6 | 6.6 | - | - |

a. The reaction solutions: 33% homogenate - 0.2 M phosphate buffer pH 7.4 (1:1 v/v).

**Table** 12. **Initial Velocities of Naproxen Formation ($v_i$) after Incubation of Dextran-Naproxen Ester Prodrugs, Varying in Molecular Size, in Pig Homogenates of Caecum and Colon with Their Content (37°C).**

| Naproxen conjugate[a] | Pig caecum homogenate[b] $v_i$ ($\mu g\ ml^{-1}/h$) | Pig Colon 1 homogenate[b] $v_i$ ($\mu g\ ml^{-1}/h$) |
|---|---|---|
| Dex T-1 (DS 2.2)[c] | 272.4 | 248.1 |
| Dex T-10 (DS 6.5) | 210.2 | 224.0 |
| Dex T-20 (DS 6.4) | 138.0 | - |
| Dex T-40 (DS 5.6) | 108.1 | - |
| Dex T-70 (DS 5.6) | 101.2 | 100.6 |
| Dex T-250 (DS 6.9) | 94.3 | 78.9 |
| Dex T-500 (DS 8.0) | 84.8 | 65.1 |

[a]The concentration of the conjugates was 20 ± 2 $mg\ ml^{-1}$

[b]The reaction solutions: 33.3% homogenate - 0.2 M phosphate buffer pH 7.4 (1:1 v/v)

[c]DS: Degree of substitution

## References cited


Gallo, J.M., E.P. Gall, W.R. Gillespie, K.S. Albert & D. Perrier (1986): *J.Clin.Pharmacol. 26,* 65-70.

Mäkelä, A.L., M. Lempiäinen & H. Ylijoki (1981): *Scand.J. Rheumatol. Suppl.39*, 15-17.

Baker, D.G. & J.L. Rabinowitz (1986): *J.Clin.Pharmacol. 26,* 2-21.

Bjarnason, I., A. So, A.J. Levi, T.J. Peters, P. Williams, G.D. Zanelli, J.M. Gumpel & B. Ansell (1984): *Lancet* (i) 1171-1173.
Hunneyball, I.M. (1986): *Pharm.Int.* 118-122.
Gray, R. & N.L. Gottlieb (1983): *Clin.Orthopaed.Rel.Res.* 235-263.
Ratcliffe, J.H., I.M. Hunneyball, C.G. Wilson, A. Smith & S.S. Davis (1987): *J.Pharm.Pharmacol. 39,* 290-295.
Ratcliffe, J.H., I.M. Hunneyball, A. Smith, C.G. Wilson & S.S. Davis (1984): *J.Pharm.Pharmacol. 36,* 431-436.
Dingle, J.T., J.L. Gordon, B.L. Hazleman, C.G. Knight, D.P. Page-Thomas, N.C. Philips, I.H. Shaw, F.J. Fildes, J.E. Oliver, G. Jones, E.H. Turner & J.S. Lowe (1978): *Nature 271,* 372-373.
de Belder, A.N. & B. Norrman (1968): *carbohydr.Res. 8,* 1-6.
Larsen, C. & M. Johansen (1985): *Int.J.Pharm. 27,* 205-218.
Casinovi, C.G., M.Framondino, G. Randazzo & F. Siani (1974): *carbohydr. Res. 36,* 67-73.
"Remington's Pharmaceutical Sciences" Sixteenth Edition (1980), Mack Publishing Company, Easton, U.S.A.
Boltze, K.-H. & H. Kreisfeld (1977): *Arzneim.-Forsch. 27,* 1300-1312.
Concilio, C. & A. Bongini (1966): *Ann.Chim. (Rome) 56,* 417-426.
Yamamoto, R., S. Fujisawa & M. Kawamura (1971): *Yakugaku Zasshi 91,* 855-862.
Vermeersch, J., F. Vandoorne, D. Permentier & E. Schacht (1985): *Bull.Soc.Chim.Belg. 94,* 591-596.
Groff, J.L., R. Cherniak & R.G. Jones (1982): *carbohydr.Res. 101,* 168-173.
Havron, A., B.-Z. Weiner & A. Zilkha (1974): *J.Med.Chem. 17,* 770-772.
Isbell, H.S., C.F. Snyder, N.B. Holt & M.R. Dryden (1953): *J.Res.-Natl.Bur.Stand. 50,* 81-86.
Brown, J.P., G.V. McGarraugh, T.M. Parkinson, R.E. Wingard, Jr. & A.B. Ouderdonk (1983): *J.Med.Chem. 26,* 1300-1307.
Thomas, P., D. Richards, L. Rogers, B.K. Evans, M.J. Dero & J. Rhodes (1985): *J.Pharm.Pharmacol. 37,* 757-758.
Parkinson, T.M., J.P. Brown & R.E. Wingard (1980): *US Patent* 4,190,716.
Parkinson, T.M., J.P. Brown & R.E. Wingard (1981): *US Patent* 4,298,595.

## Claims

1. Compounds of the formula 1

$$PS - O - A - (CH_2)_n - B - D \qquad (1)$$

wherein PS-O represents an alkoxide residue of any of the free hydroxy groups of a polysaccharide (PS-OH) compound with molecular weight ($M_w$) of from 40,000 to 5,000,000 selected from dextran, carboxymethyl dextran, diethylaminoethyl dextran, glycogen, pullullan, agarose, cellulose, chitosan, chitin and carrageenan,

A is a carbonyl group or absent,

n is zero or a positive integer from 1 to 14,

B is oxygen, a carbonyl group, NR wherein R is hydrogen or straight- or branched-chain $C_{1-8}$ alkyl, or B is absent, and

D is

(i) a group of the formula:

$$R_1 - CO - \qquad (1_1)$$

wherein $R_1$-CO- represents the acyl residue of a carboxylic acid drug ($R_1COOH$) used in the treatment of inflammatory disorders; or

(ii) a group of the formula:

$$R_2 - CO - \qquad (1_2)$$

wherein $R_2$-O- refers to the C-21 alkoxide residue of a known anti-inflammatory steroid ($R_2$-OH) or an alkoxide residue of any other drug or medicament which is used in the treatment of inflammatory disorders and which contains a hydroxy functional group;

with the proviso that when PS-O is the alkoxide residue of dextran, A is absent, n is 0, and B is absent, then $R_1$-CO- is different from the acyl residue of acetylsalicylic acid; and with the further proviso that compounds having peroxo (-O-O-) moieties are excluded;

and non-toxic pharmaceutically acceptable acid addition salts thereof;

and non-toxic pharmaceutically acceptable cation salts thereof.

2. Compounds according to claim 1 wherein $R_1$-CO- is derived from a compound selected from the group consisting of :
   Sulindac™
   Naproxen™
   Fenoprofen™
   Ibuprofen™
   Ketoprofen™
   Indoprofen™
   Flurbiprofen™
   Mefenamic acid™
   Flufenamic acid™
   Meclofenamic acid™
   Fluprofen™
   Fenclofenac™
   Lonazolac™
   Fentufen™
   Carprofen™
   Loxoprofen™
   5-aminosalicylic acid
   Salazosulfapyridine™
   Azodisal sodium™
   Penicillamin™
   Chlorambucil™
   Melphalan™
   Gold sodium thiomalate
   Furosemide™.

3. Compound according to claim 1 wherein $R_2$-O- is derived from a compound selected from the group consisting of :
   Hydrocortisone
   Betamethasone™
   Dexamethasone™
   Prednisolone
   Triamcinolone™
   Methylprednisolone
   Triamcinolone acetonide™
   Aurothioglucose™
   Hydroxychloroquine
   Amodiaquin™
   Quinine™.

4. A pharmaceutical composition for parenteral administration comprising pharmaceutically acceptable excipients including liposomes and microspheres and a pharmaceutically effective amount of a compound according to any of claims 1-3.

5. A pharmaceutical composition for oral administration comprising pharmaceutically acceptable excipients and a pharmaceutically effective amount of a compound according to any of claims 1-3.

6. A pharmaceutical composition for intra-articular administration comprising pharmaceutically acceptable excipients and a pharmaceutically effective amount of a compound according to any of claims 1-3.

7. A pharmaceutical composition for subcutaneous administration comprising pharmaceutically acceptable excipients and a pharmaceutically effective amount of a compound according to any of claims 1-3.

8. A pharmaceutical composition for intra-muscular administration comprising pharmaceutically acceptable excipients and a pharmaceutically effective amount of a compound according to any of claims 1-3.

9. A pharmaceutical composition for extra-dural administration comprising pharmaceutically acceptable excipients and a pharmaceutically effective amount of a compound according to any of claims 1-3.

10. A process for preparing a compound of the formula 1 as defined in claim 1 comprising

a. reacting the carboxylic acid compound of the formula E or a salt thereof

$$\text{ligand - COOH} \quad (E)$$

wherein ligand-COOH represents any of the carboxylic acid structures given by the formulas F, G, H, J

$$R_1 - COOH \quad (F)$$
$$R_1 - CO - O - (CH_2)_n - COOH \quad (G)$$
$$R_1 - CO - NR - (CH_2)_n - COOH \quad (H)$$
$$R_2 - O - CO - (CH_2)_n - COOH \quad (J)$$

wherein $R_1$-CO-, n, R and $R_2$-O- are defined as above in connection with formula 1, with a compound having the formula K

$$PS - OH \quad (K)$$

wherein PS-O is as defined above in connection with Formula 1; or

b. reacting a compound of formula K with an acid chloride of formula L

$$\text{ligand - COCl} \quad (L)$$

wherein ligand is as defined in connection with formula E; or

c. reacting a compound of formula M or a salt thereof

$$PS - O - A - (CH_2)_n - COOH \quad (M)$$

wherein PS-O, A and n are as defined in connection with formula 1, with a compound having the formula P

$$R_2W \quad (P)$$

wherein $R_2$ ($R_2$-O) is as defined in connection with formula 1 and W is a suitable leaving group; or

d. reacting a compound of formula K with a compound having the formula S

$$R_2 - O - COCl \quad (S)$$

wherein $R_2$-O is as defined in connection with formula 1.

11. Use of a compound according to any of claims 1-3 for preparing a pharmaceutical composition for use in the treatment of inflammatory bowel diseases such as ulcerative colitis or Morbus Crohn.

12. Use of a compound according to any of claims 1-3 for preparing an oral pharmaceutical composition for use in the treatment of inflammatory diseases occurring in the terminal ileum, caecum or colon.

**Patentansprüche**

1. Verbindungen der Formel 1

$$PS - O - A - (CH_2)_n - B - D \quad (1)$$

in der PS-O einen Alkoxidrest einer beliebigen freien Hydroxylgruppe einer Polysaccharid-(PS-OH)-Verbindung mit einem Molekulargewicht ($M_w$) von 40 000 bis 5 000 000 bedeutet, welche ausgewählt ist aus Dextran, Carboxymethyldextran, Diethylaminoethyldextran, Glycogen, Pullullan, Agarose, cellulose, Chitosan, Chitin und Carrageenan,

A eine carbonylgruppe bedeutet oder nicht vorliegt,

n Null oder eine positive ganze Zahl von 1 bis 14 bedeutet,

B ein Sauerstoffatom, eine Carbonylgruppe oder einen Rest der Formel NR bedeutet, in der R ein Wasserstoffatom oder einen gerad- oder verzweigtkettigen $C_{1-8}$-Alkylrest bedeutet, oder B nicht vorliegt, und

D

(i) einen Rest der Formel:

$$R_1 - CO - \quad (1_1)$$

in der $R_1$-CO- einen Acylrest eines carbonsäuregruppen-haltigen Arzneistoffes ($R_1$-COOH) darstellt, der zur Behandlung von entzündlichen Erkrankungen verwendet wird; oder

(ii) einen Rest der Formel:

$$R_2 - O - \quad (1_2)$$

bedeutet, in der $R_2$-O- einen C-21-Alkoxidrest eines bekannten entzündungshemmenden Steroids ($R_2$-OH) oder einen Alkoxidrest eines anderen Arzneistoffs oder Medikaments bedeutet, der/das bei der Behandlung entzündlicher Erkrankungen vewendet wird und eine Hydroxygruppe als funktionelle Gruppe enthält;

mit der Maßgabe, daß, wenn PS-O der Alkoxidrest von Dextran ist, A nicht vorliegt, n 0 ist und B nicht

vorliegt, der Rest $R_1$-CO- dann vom Acylrest der Acetylsalicylsäure verschieden ist; und mit der weiteren Maßgabe, daß Verbindungen mit Peroxo (-O-O-)-Einheiten ausgenommen sind; und nicht-toxische pharmazeutisch verträgliche Säureadditionssalze davon;

und nicht-toxische pharmazeutisch verträgliche Kationensalze davon.

**2.** Verbindungen nach Anspruch 1, wobei $R_1$-CO abgeleitet ist von einer Verbindung, ausgewählt aus der Gruppe:

Sulindac™, Naproxen™, Fenoprofen™, Ibuprofen™, Ketoprofen™, Indoprofen™, Flurbiprofen™, Mefenamic acid™, Flufenamic acid™, Meclofenamic acid™, Fluprofen™, Fenclofenac™, Lonazolac™, Fenbufen™, Carprofen™, Loxoprofen™, 5-Aminosalicylsäure, Salazosulfapyridine™, Azodisal sodium™, Penicillamin™, Chlorambucil™, Melphalan™, Gold-Natrium-Thiomalat, Furosemide™.

**3.** Verbindungen nach Anspruch 1, wobei $R_2$-O abgeleitet ist von einer Verbindung, ausgewählt aus der Gruppe:

Hydrocortison, Betamethasone™, Dexamethasone™, Prednisolon, Triamcinolone™, Methylprednisolon, Triamicinolone acetonide™, Aurothioglucose™, Hydroxychloroquin, Amodiaquin™, Quinine™ (Chinin).

**4.** Arzneimittel zur parenteralen Verabreichung, umfassend pharmazeutisch verträgliche Excipienten, einschließlich Liposomen und Mikrokügelchen, und eine pharmazeutisch wirksame Menge einer Verbindung nach einem der Ansprüche 1 bis 3.

**5.** Arzneimittel zur oralen Verabreichung, umfassend pharmazeutisch verträgliche Excipienten und eine pharmazeutisch wirksame Menge einer Verbindung nach einem der Ansprüche 1 bis 3.

**6.** Arzneimittel zur intraartikulären Verabreichung, umfassend send pharmazeutisch Verträgliche Excipienten und eine pharmazeutisch wirksame Menge einer Verbindung nach einem der Ansprüche 1 bis 3.

**7.** Arzneimittel zur subcutanen Verabreichung, umfassend pharmazeutisch verträgliche Excipienten und eine pharmazeutisch wirksame Menge einer Verbindung nach einem der Ansprüche 1 bis 3.

**8.** Arzneimittel zur intramuskulären Verabreichung, umfassend pharmazeutisch verträgliche Excipienten und eine pharmazeutisch wirksame Menge einer verbindung nach einem der Ansprüche 1 bis 3.

**9.** Arzneimittel zur extraduralen Verabreichung, umfassend pharmazeuticch verträgliche Excipienten und eine pharmazeutisch wirksame Menge einer Verbindung nach einem der Ansprüche 1 bis 3.

**10.** Verfahren zur Herstellung einer Verbindung der Formel 1 gemäß Anspruch 1, umfassend:

a. Umsetzung der Carbonsäureverbindung der Formeln E oder eines Salzes davon

$$\text{Ligand - COOH} \qquad (E)$$

in der Ligand-COOH eine der durch die Formeln F, G, H, J angegebenen Carbonsäurestrukturen darstellt:

$$R_1 - COOH \qquad (F)$$

$$R_1 - CO - O - (CH_2)_n - COOH \qquad (G)$$

$$R_1 - CO - NR - (CH_2)_n - COOH \qquad (H)$$

$$R_2 - O - CO - (CH_2)_n - COOH \qquad (J)$$

in denen $R_1$-CO-, n, R und $R_2$-O- wie vorstehend im Zusammenhang mit Formel 1 definiert sind, mit einer Verbindung der Formel K

$$PS - OH \qquad (K)$$

in dar PS-O wie vorstehend im zusammenhang mit Formel 1 definiert ist; oder

b. Umsetzung einer Verbindung der Formel K mit einen Säurechlorid der Formel L

$$\text{Ligand - COCl} \qquad (L)$$

in der Ligand wie vorstehend im Zusammenhang mit Formel E definiert ist; oder

c. Umsetzung einer Verbindung der Formel M oder eines Salzes davon

$$PS - O - A - (CH_2)_n - COOH \qquad (M)$$

in der PS-O, A und n wie vorstehend im Zusammenhang mit Formel 1 definiert sind, mit einer Verbindung der Formel P

$$R_2W \qquad (P)$$

in der $R_2$ ($R_2$-O) wie vorstehend im Zusammenhang mit Formel 1 definiert ist und W eine geeignete Austrittsgruppe darstellt; oder

d. Umsetzung einer Verbindung der Formel K mit einer Verbindung der Formel S

$$R_2 - O - COCl \qquad (S)$$

in der $R_2$-O wie vorstehend im Zusammenhang mit Formel 1 definiert ist.

**11.** Verwendung einer Verbindung nach einem der Ansprüche 1 bis 3, zur Herstellung eines Arzneimittels zur Verwendung bei der Behandlung von entzündlichen Darmerkrankungen, wie ulceröse Colitis oder Morbus Crohn.

**12.** Verwendung einer Verbindung nach einem der Ansprüche 1 bis 3 zur Herstellung eines Arzneimittels zur oralen Verabreichung zur Verwendung bei der Behandlung von entzündlichen Erkrankungen, die im Endileum, Caecum oder Colon auftreten.

## Revendications

**1.** Composé de formule 1:

$$PS - O - A - (CH_2)_n - B - D \qquad (1)$$

dans laquelle:

PS-O représente un résidu alkoxyde de l'un quelconque des groupes hydroxy libres d'un polysaccharide (PS-OH) ayant un poids moléculaire ($M_w$) compris entre 40 000 et 5 000 000, choisi parmi le dextrane, le carboxyméthyldextrane, le diéthylaminoéthyldextrane, le glycogène, le pullullane, l'agarose, la cellulose, la chitosane, la chitine et la carragénane;

A est un groupe carbonyle ou est absent;

n vaut zéro ou un entier positif compris entre 1 et 14;

B est l'oxygène, un groupe carbonyle, un groupe NR dans lequel R est un atome d'hydrogène ou un groupe alkyle en $C_1$-$C_8$, linéaire ou ramifié, ou B est absent; et

D est

(i) un groupe de formule:

$$R_1 - CO - \qquad (1_1)$$

dans laquelle:

$R_1$-CO- représente le résidu acyle d'un médicament acide carboxylique ($R_1$-COOH) utilisé dans le traitement de troubles inflammatoires;

ou

(ii) un groupe de formule:

$$R_2 - O - \qquad (1_2)$$

dans laquelle:

$R_2$-O- se réfère au résidu alkoxyde en position $C_{21}$ d'un anti-inflammatoire stéroïdal connu ($R_2$-OH) ou un résidu alkoxyde de n'importe quel autre médicament qui est utilisé dans le traitement des troubles inflammatoires et qui contient un groupe fonctionnel hydroxy;

à condition que, lorsque PS-O est le résidu alkoxyde du dextrane, A est absent, n vaut 0, et B est absent, alors $R_1$-CO- est différent du résidu acyle de l'acide acétylsalicylique; et à l'autre condition que les composés ayant des liaisons peroxo (-O-O-) sont exclus;

ainsi que les sels d'addition d'acides non toxiques pharmaceutiquement acceptables; et leurs sels cationiques non toxiques pharmaceutiquement acceptables.

**2.** Composés selon la revendication 1, dans lesquels $R_1$-CO- est dérivé d'un composé choisi dans le groupe consistant en:

Sulindac™
Naproxen™
Fenoprofen™
Ibuprofen™
Ketoprofen™
Indoprofen™
Flurbiprofen™
Acide méfénamique™
Acide flufénamique™

Acide méclofénamique™
Fluprofen™
Fenclofenac™
Lonazolac™
Fenbufen™
Carprofen™
Loxoprofen™
Acide 5-aminosalicylique
Salazosulfapyridine™
Azodisal sodium™
Penicillamin™
Chlorambucil™
Melphalan™
Thiomalate d'or-sodium
Furosemide™

**3.** Composés selon la revendication 1, dans lesquels $R_2$-O- est dérivé d'un composé choisi dans le groupe consistant en:

Hydrocortisone
Betamethasone™
Dexamethasone™
Prednisolone
Triamcinolone™
Méthylprednisolone
Triamcinolone acétonide™
Aurothioglucose™
Hydroxychloroquine
Amodiaquin™
Quinine™

**4.** Une composition pharmaceutique pour administration parentérale comprenant des excipients pharmaceutiquement acceptables incluant les liposomes et des microsphères et une quantité pharmaceutiquement efficace d'un composé selon l'une quelconque des revendications 1 à 3.

**5.** Une composition pharmaceutique pour administration orale comprenant des excipients pharmaceutiquement acceptables et une quantité pharmaceutiquement efficace d'un composé selon l'une quelconque des revendications 1 à 3.

**6.** Une composition pharmaceutique pour administration intraarticulaire comprenant des excipients pharmaceutiquement acceptables et une quantité pharmaceutiquement efficace d'un composé selon l'une quelconque des revendications 1 à 3.

**7.** Une composition pharmaceutique pour administration sous-cutanée comprenant des excipients pharmaceutiquement acceptables et une quantité pharmaceutiquement efficace d'un composé selon l'une quelconque des revendications 1 à 3.

**8.** Une composition pharmaceutique pour administration intramusculaire comprenant des excipients pharmaceutiquement acceptables et une quantité pharmaceutiquement efficace d'un composé selon l'une quelconque des revendications 1 à 3.

**9.** Une composition pharmaceutique pour administration extra-durale comprenant des excipients pharmaceutiquement acceptables et une quantité pharmaceutiquement efficace d'un composé selon l'une quelconque des revendications 1 à 3.

**10.** Procédé de préparation d'un composé de formule 1 tel que défini dans la revendication 1 comprenant les étapes de:

a) réaction d'un composé acide carboxylique de formule E, ou un de ses sels:

ligand - COOH (E)

dans laquelle:

ligand-COOH représente n'importe laquelle des structures acide carboxylique données par les formules F, G, H, J:

$$R_1 - COOH \qquad (F)$$
$$R_1 - CO - O - (CH_2)_n - COOH \qquad (G)$$
$$R_1 - CO - NR - (CH_2)_n - COOH \qquad (H)$$
$$R_2 - O - CO - (CH_2)_n - COOH \qquad (J)$$

dans lesquelles $R_1$-CO-, n, R et $R_2$-O- sont définis tels que ci-dessus dans la formule 1, avec un composé ayant la formule K:

$$PS - OH \qquad (K)$$

dans laquelle:
PS-O est tel que défini ci-dessus en relation avec la formule 1;
ou
b) réaction d'un composé de formule K avec un chlorure d'acide de formule L:

$$ligand - COCl \qquad (L)$$

dans laquelle:
ligand est tel que défini en relation avec la formule E;
ou
c) réaction d'un composé de formule M ou d'un de ses sels:

$$PS - O - A - (CH_2)_n - COOH \qquad (M)$$

dans laquelle:
PS-O, A et n sont tels que définis en relation avec la formule 1,
avec un composé de formule P:

$$R_2W \qquad (P)$$

dans laquelle:
$R_2$ ($R_2$-O) est défini an relation avec la formule 1; et W est un groupe partant approprié;
ou
d) réaction d'un composé de formule K avec un composé de formule S:

$$R_2 - O - COCl \qquad (S)$$

dans laquelle:
$R_2$-O est tel que défini en relation avec la formule 1.

**11.** Utilisation d'un composé selon l'une quelconque des revendications 1 à 3 pour la préparation d'une composition pharmaceutique detinée au traitement des troubles inflammatoires de l'intestin tels que la colite avec ulcération ou la maladie de Crohn.

**12.** Utilisation d'un composé selon l'une quelconque des revendications 1 à 3 pour la préparation d'une composition pharmaceutique orale pour l'emploi dans le traitement des troubles inflammatoires ayant lieu dans la partie terminale de l'iléon, dans le caecum ou dans le colon.

# Fig. 1

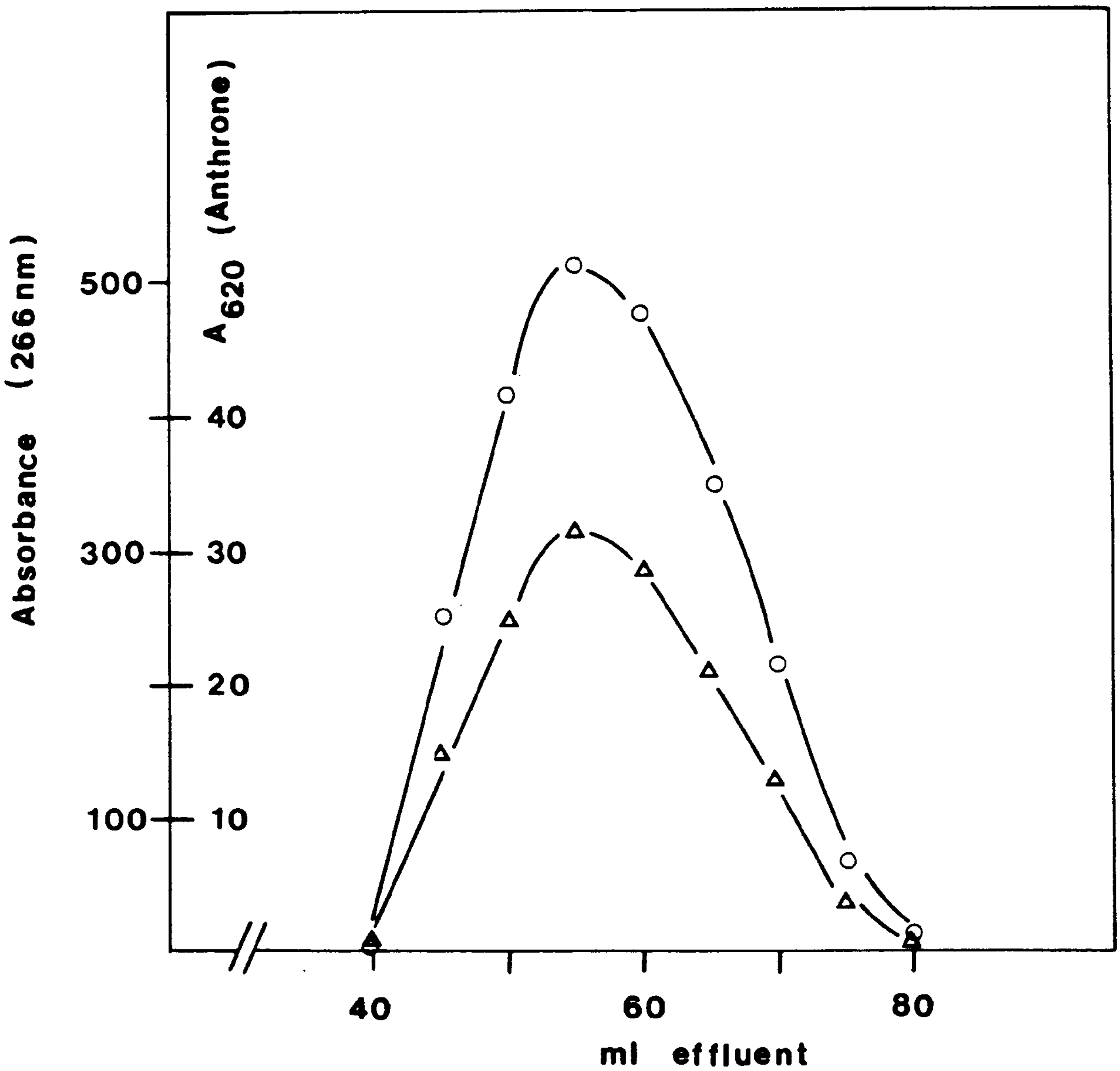
Absorbance (266 nm)
$A_{620}$ (Anthrone)
500
300
100
40
30
20
10
40
60
80
ml effluent

# Fig. 2

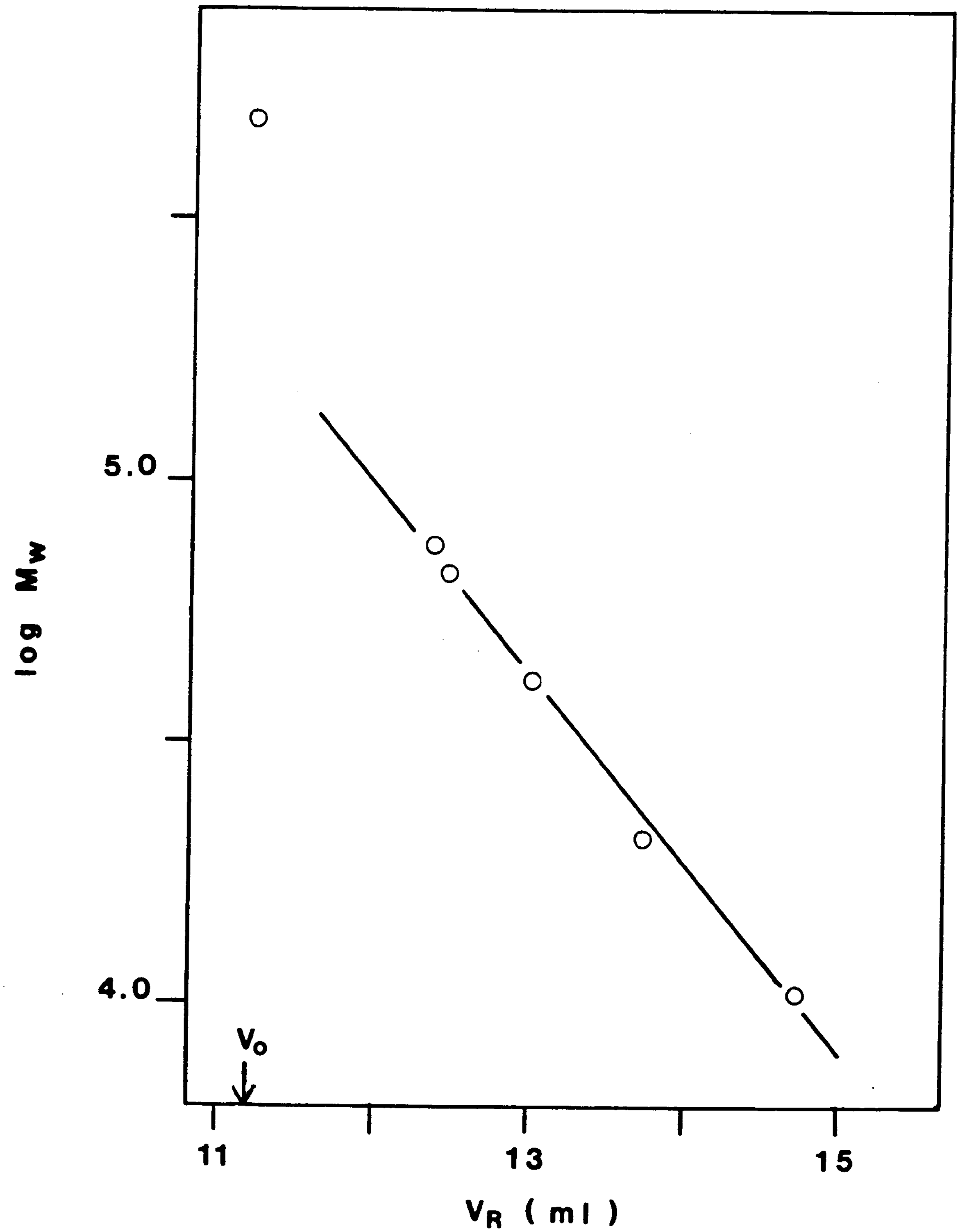

Fig. 3

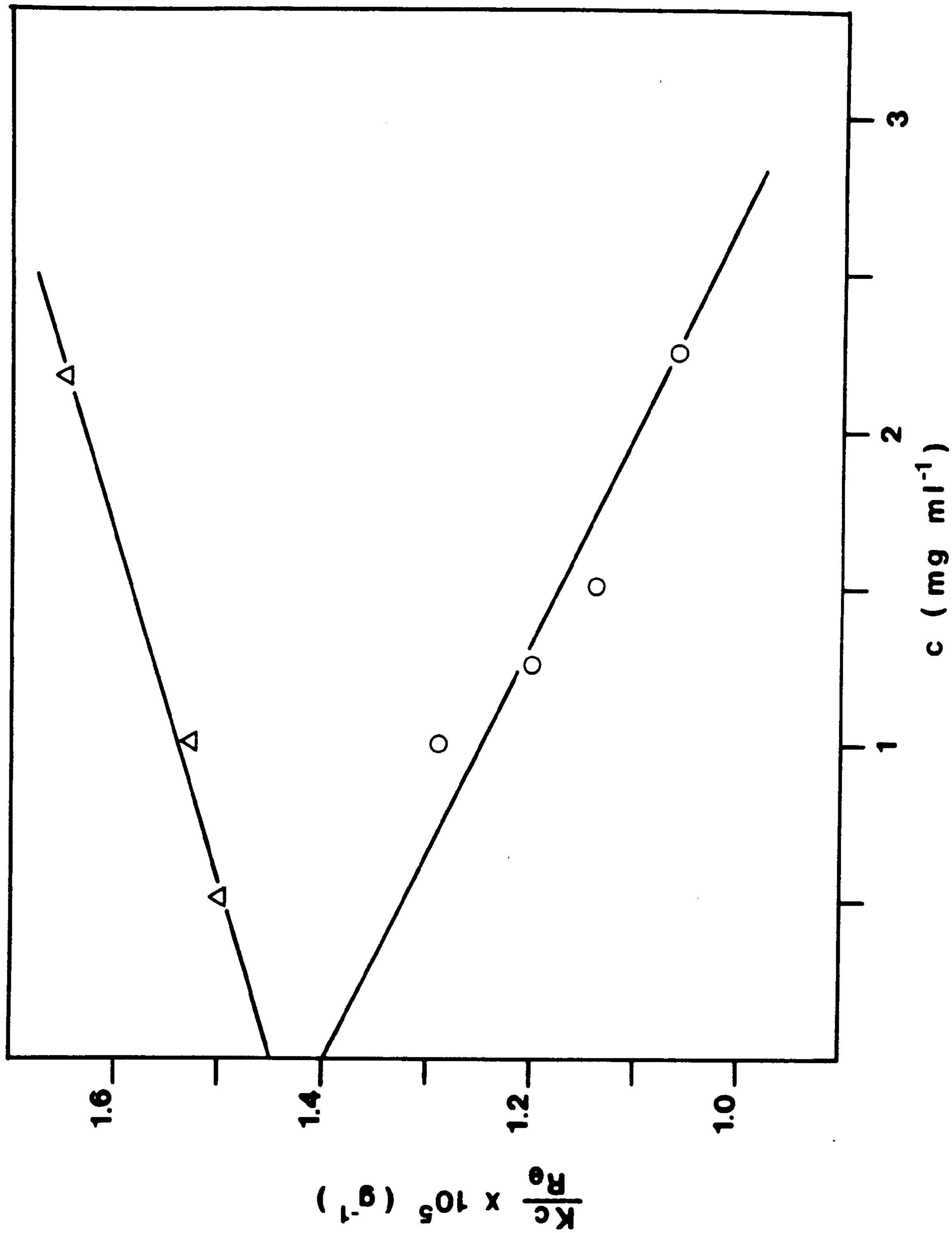

# Fig. 4

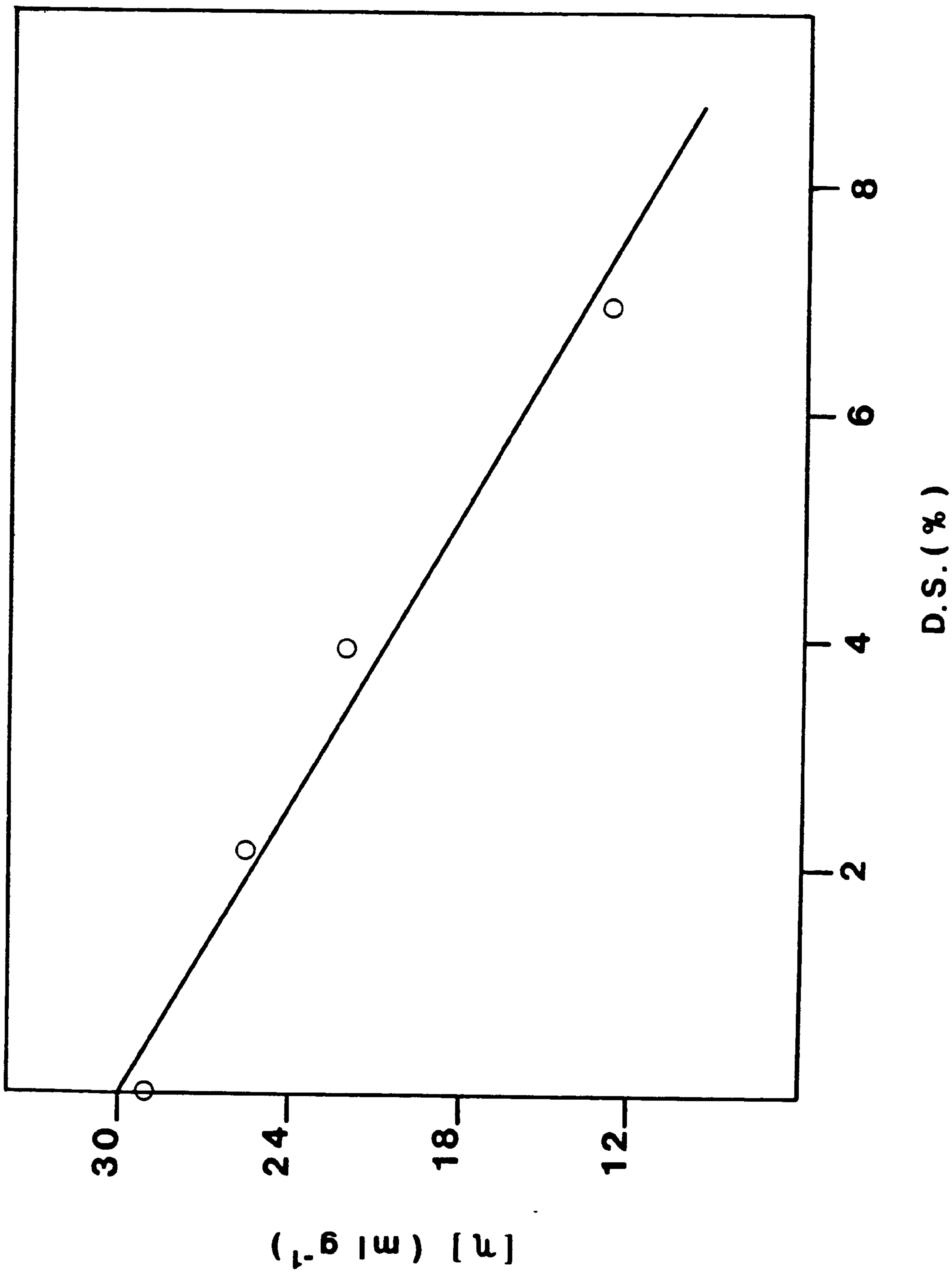

# Fig. 5

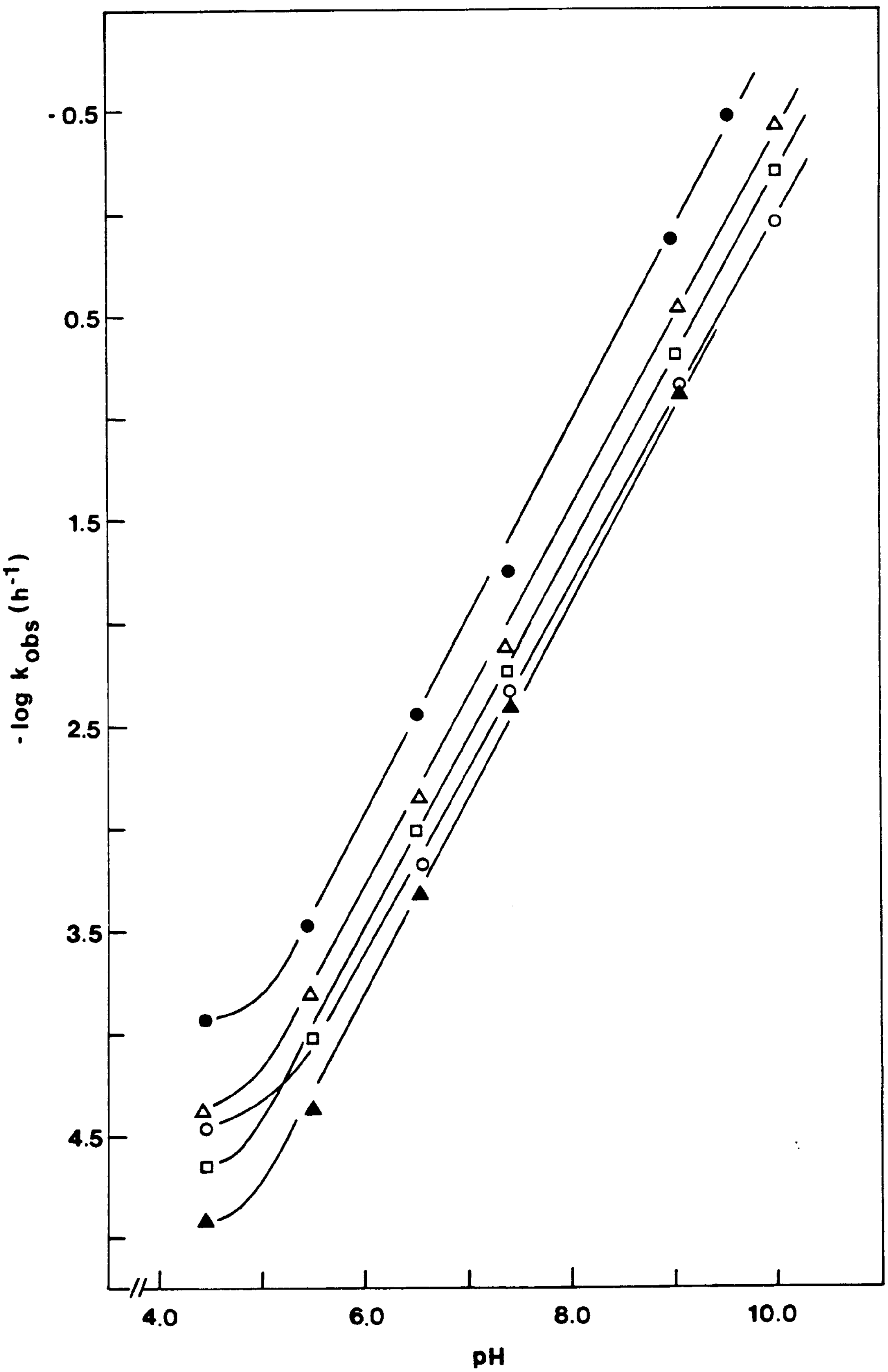

# Fig. 6

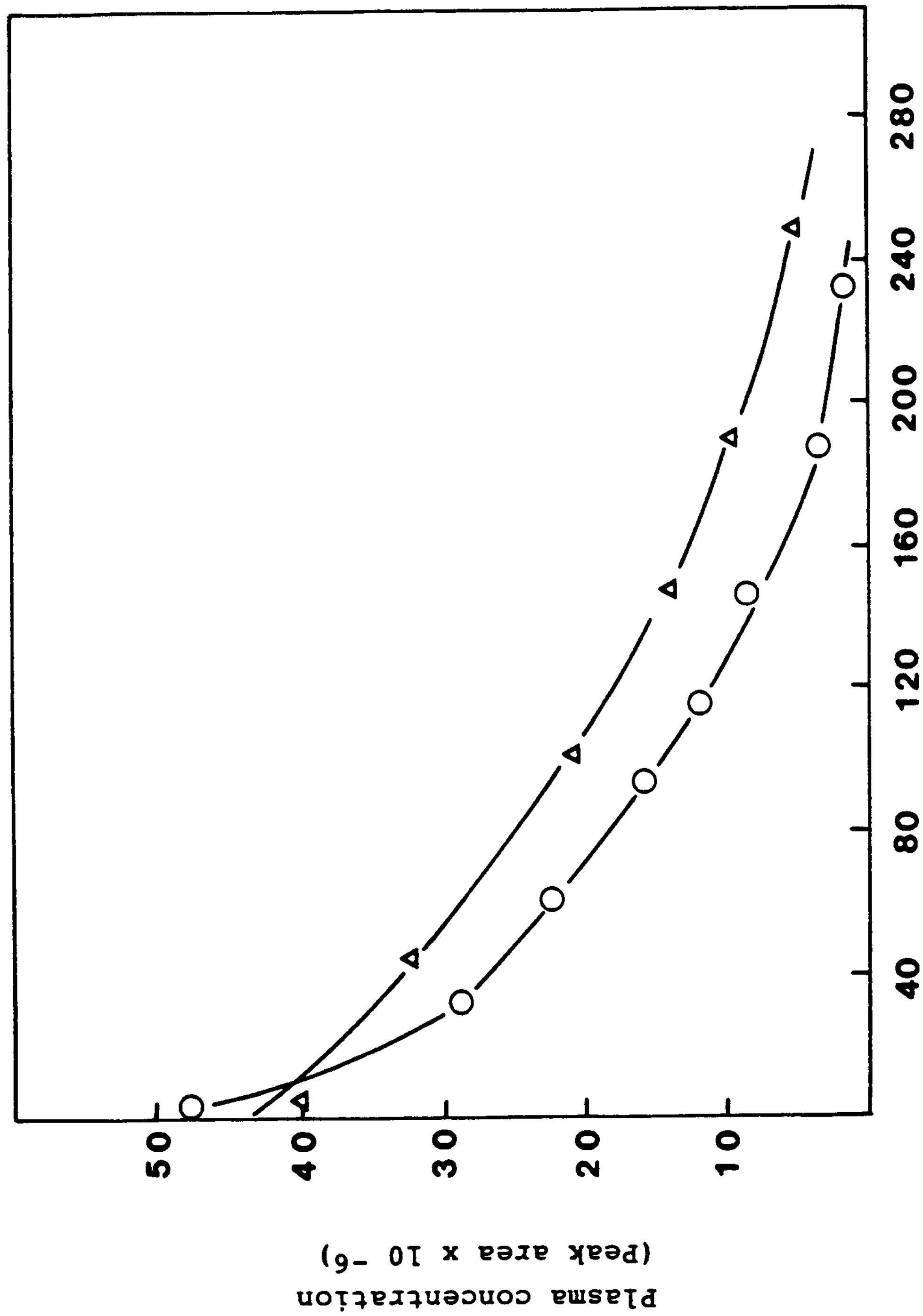
Plasma concentration
(Peak area x 10 -6)
50
40
30
20
10
40
80
120
160
200
240
280

# Fig. 7

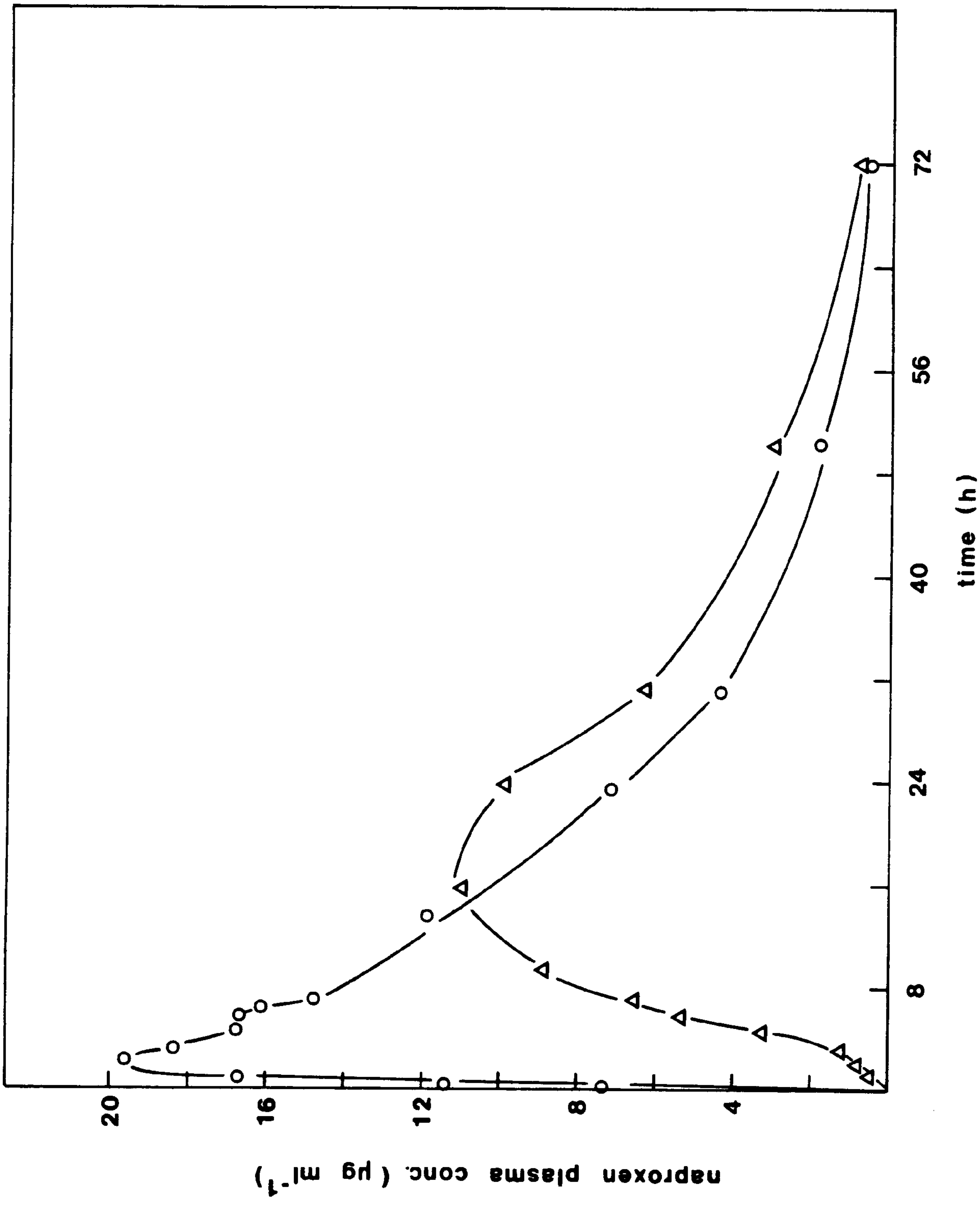

# Fig. 8

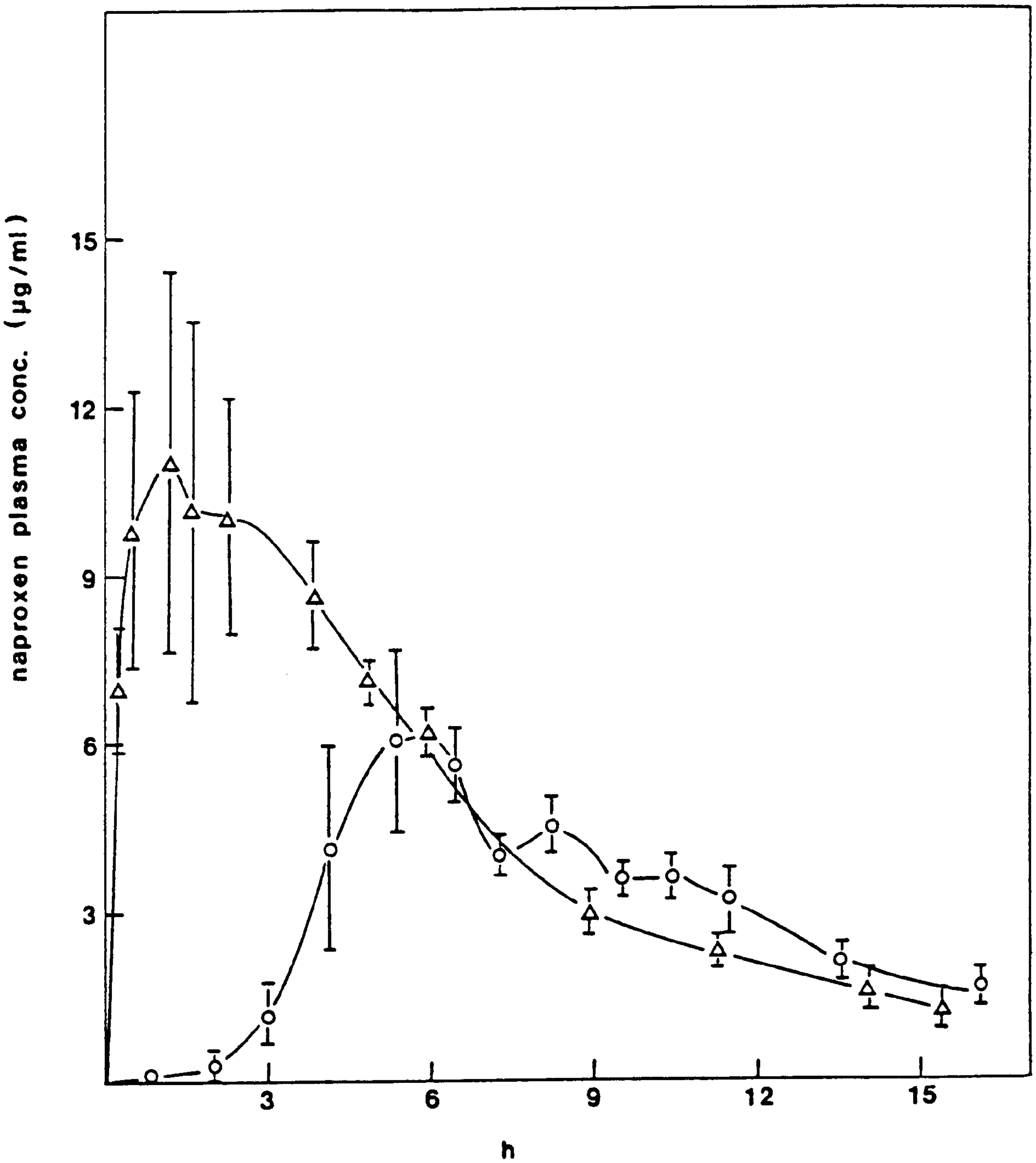

# Fig. 9